# EUROPEAN PATENT APPLICATION

(11) **EP 4 717 715 A1**
(43) Date of publication of application: **01.04.2026**
(21) Application number: 24810355.8
(22) Date of filing: 20.05.2024
(51) Int. Cl.: C07K 19/00, C07K 14/015, C07K 7/08, C07K 7/06, C12N 15/864, A61K 48/00

(54) **ADENO-ASSOCIATED VIRUS CAPSID PROTEIN, ADENO-ASSOCIATED VIRUS CONTAINING SAME, VECTOR AND USE THEREOF**

(30) Priority: 23.05.2023 WO PCT/CN2023/095837
(71) Applicant: InnoVec Biotherapeutics, Beijing 100094 (CN)
(72) Inventor: WANG, Cheng, Beijing 100094 (CN); FU, Yingying, Beijing 100094 (CN); LI, Wei, Beijing 100094 (CN)
(74) Representative: E. Blum & Co. AG
(86) International application number: PCT/CN2024/094226
(87) International publication number: WO 2024/240113

(57) **Abstract**

Provided are an adeno-associated virus capsid protein, an adeno-associated virus containing same, a vector, and the use thereof. Specifically, provided is an adeno-associated virus capsid protein comprising a targeting peptide. The targeting peptide contains one or more of the following sequences: (i) sequence AAX₁X₂X₃X₄X₅X₆X₇X₈AA; and (ii) sequence NG. Further provided are a targeting peptide, an adeno-associated virus, a transgenic delivery vector, a pharmaceutical composition, etc. Compared with a wild-type adeno-associated virus, the provided adeno-associated virus capsid protein and the adeno-associated virus containing same have improved cell infection efficiency in vivo and/or in vitro, have a good targeting effect on eyes, and can be used as a delivery vector for the treatment of eye-related diseases.

## Description

### PRIORITY AND RELATED APPLICATIONS

The present disclosure claims the benefit of a priority of the PCT International Application No. PCT/CN2023/095837, filed on May 23, 2023 and entitled "ADENO-ASSOCIATED VIRUS CAPSID PROTEIN, ADENO-ASSOCIATED VIRUS CONTAINING SAME, VECTOR AND USE THEREOF", the entire contents of which including the appendices are incorporated herein by reference.

### TECHNICAL FIELD

The present disclosure belongs to the technical field of viral vectors, and specifically relates to an adeno-associated virus capsid protein, an adeno-associated virus comprising the same, a vector thereof, and use thereof.

### BACKGROUND

With the increasing number of market-approved gene therapy drugs, the focus of development has shifted towards improving the delivery efficiency and safety of gene delivery vectors, including the recombination adeno-associated virus (rAAV) vectors. For example, in 2017, "Luxturna" - an adeno-associated virus type 2 (AAV2)-based gene therapy drug - produced by Spark Therapeutics was approved by the Food and Drug Administration (FDA) for treatment of Leber Congenital Amaurosis (LCA) caused by RPE65 mutations. Adeno-associated virus (AAV) has low pathogenicity and has the ability to stably express proteins in various tissues and organs for a long time. However, wild-type AAV serotypes typically infect multiple tissues/organs in mammals in a broad spectrum manner and have an extensive tissue targeting capacity, leading to delivery of genes to off-target sites, thereby exacerbating adverse effects.

The tissue tropism and cell transduction efficiency of AAV vectors are mainly determined by their capsids. To achieve better therapeutic effects, it is desirable to obtain AAV vectors specific to organs (e.g. eyes) through appropriate modifications of the AAV capsid protein (Cap). This can typically be accomplished by inserting a polypeptide composed of 7 to 20 amino acids into the AAV capsid protein by the site-directed mutagenesis technology. For example, a peptide L14 composed of 14 amino acids (specifically, QAGTFALRGDNPQG, SEQ ID NO: 66; containing an RGD structure) is inserted after the 587^{th} amino acid in the coding sequence of the capsid protein in AAV2 to obtain vectors targeting β1-integrin-positive tumor cells (Girod et al., (1999), Nat. Med. 5, 1052-1056).

However, the difference in polypeptides inserted into the AAV capsid protein makes the effects thereby achieved unpredictable. In view of this, there is still a need in this field to develop new modified AAV capsid proteins and to apply them to AAV vectors as effective gene delivery vectors.

### SUMMARY

### Technical Problem

In view of the above problems existing in the prior art, an object of the present disclosure is to provide a targeting peptide targeting eyes used in an AAV capsid protein, and an AAV capsid protein containing the targeting peptide, an AAV, and an AAV vector, so as to improve the targeting capability of the AAV/AAV vector as a gene delivery vector to eyes.

### Solution to Problem

To achieve better therapeutic effects, the present disclosure designs 15 modifications by inserting several amino acids after the 587^{th} amino acids in AAV2 or by substituting for the 586^{th} and 587^{th} amino acids in AAV2, in the hope of obtaining novel adeno-associated virus vectors with a high retinal transduction efficiency.

According to the first aspect of the present disclosure, there is provided an adeno-associated virus capsid protein, comprising a targeting peptide, wherein the targeting peptide comprises one or more of the following sequences:
(i) a sequence AAX₁X₂X₃X₄X₅X₆X₇X₈AA; and
(ii) a sequence NG;
wherein in the sequence AAX₁X₂X₃X₄X₅X₆X₇X₈AA:
(a) X₁ is A;
(b) X₂ is R or G;
(c) X₃ is G or N;
(d) X₄ is D, S or G;
(e) X₅ is L or R;
(f) X₆ is A, G, Q or a deletion;
(g) X₇ is T, R, N, A, H or a deletion; and
(h) X₈ is I, L, P, A or a deletion.

In some embodiments, in the sequence AAX₁X₂X₃X₄X₅X₆X₇X₈AA:
(a) X₁ is A;
(b) X₂ is R;
(c) X₃ is G;
(d) X₄ is D or S;
(e) X₅ is L;
(f) X₆ is A, G, Q or a deletion;
(g) X₇ is T, R, N, A or a deletion; and
(h) X₈ is I, L, P or a deletion.

In some specific embodiments, the targeting peptide comprises an amino acid sequence as set forth in any one of SEQ ID NOs: 1 to 14 and the sequence NG.

In some preferred embodiments, the targeting peptide comprises an amino acid sequence as set forth in any one of SEQ ID NOs: 2, 8, 11, 12, 13, and 14 and the sequence NG.

In some more preferred embodiments, the targeting peptide comprises an amino acid sequence as set forth in any one of SEQ ID NOs: 8, 13, and 14 and the sequence NG.

In some embodiments, the adeno-associated virus is one or more selected from AAV1, AAV2, AAV3, AAV4, AAV5, AAV6, AAV7, AAV8, AAV9, rh.10, rh.39, rh.43, and rh.74.

In some preferred embodiments, AAV comprises AAV2.

In some embodiments, AAV2 comprises AAV2 VP1 or a variant thereof;
In some preferred embodiments, the amino acid sequence of AAV2 VP1 is as set forth in SEQ ID NO: 60.

In some preferred embodiments, the variant of AAV2 VP1 comprises a variant in which proline (P) at position 34 is mutated to alanine (A) relative to the amino acid sequence as set forth in SEQ ID NO: 60.

In some specific embodiments, the targeting peptide is inserted at a position corresponding to the 587^{th} amino acid in AAV2 VP1 or a variant thereof.

In some specific embodiments, the targeting peptide substitutes for the 586^{th} amino acid and the 587^{th} amino acid in AAV2 VP1 or a variant thereof.

In some preferred embodiments, the adeno-associated virus capsid protein comprises an amino acid sequence as set forth in any one of SEQ ID NOs: 15 to 29 and 65, or an amino acid sequence having at least 80%, 85%, 90%, 95%, 97% or 99% identity to the amino acid sequence as set forth in any one of SEQ ID NOs: 15 to 29 and 65.

According to the second aspect of the present disclosure, there is provided a targeting peptide comprising one or more of the following sequences:
(i) a sequence AAX₁X₂X₃X₄X₅X₆X₇X₈AA; and
(ii) a sequence NG;
wherein in the sequence AAX₁X₂X₃X₄X₅X₆X₇X₈AA:
(a) X₁ is A;
(b) X₂ is R or G;
(c) X₃ is G or N;
(d) X₄ is D, S or G;
(e) X₅ is L or R;
(f) X₆ is A, G, Q or a deletion;
(g) X₇ is T, R, N, A, H or a deletion; and
(h) X₈ is I, L, P, A or a deletion.

In some optional embodiments, in the sequence AAX₁X₂X₃X₄X₅X₆X₇X₈AA:
(a) X₁ is A;
(b) X₂ is R;
(c) X₃ is G;
(d) X₄ is D or S;
(e) X₅ is L;
(f) X₆ is A, G, Q or a deletion;
(g) X₇ is T, R, N, A or a deletion; and
(h) X₈ is I, L, P or a deletion.

In some preferred embodiments, the targeting peptide comprises an amino acid sequence as set forth in any one of SEQ ID NOs: 1 to 14 and the sequence NG.

In some more preferred embodiments, the targeting peptide comprises an amino acid sequence as set forth in any one of SEQ ID NOs: 2, 8, 11, 12, 13, and 14 and the sequence NG.

In some further preferred embodiments, the targeting peptide comprises an amino acid sequence as set forth in any one of SEQ ID NOs: 8, 13 and 14.

According to the third aspect of the present disclosure, there is provided a polynucleotide encoding the adeno-associated virus capsid protein according to the first aspect of the present disclosure, or encoding the targeting peptide according to the second aspect of the present disclosure.

According to the fourth aspect of the present disclosure, there is provided an adeno-associated virus, comprising the adeno-associated virus capsid protein according to the first aspect of the present disclosure.

In some embodiments, the adeno-associated virus further comprises a transgene.

In some optional embodiments, the transgene is a therapeutic transgene, a prophylactic transgene or a diagnostic transgene.

According to the fifth aspect of the present disclosure, there is provided a transgene delivery vector comprising the adeno-associated virus according to the fourth aspect of the present disclosure.

According to the sixth aspect of the present disclosure, there is provided a pharmaceutical composition comprising:
(a) the adeno-associated virus according to the fourth aspect of the present disclosure or the transgene delivery vector according to the fifth aspect of the present disclosure; and optionally
(b) a pharmaceutically acceptable carrier.

According to the seventh aspect of the present disclosure, there is provided use of the adeno-associated virus according to the fourth aspect of the present disclosure, the transgene delivery vector according to the fifth aspect of the present disclosure, or the pharmaceutical composition according to the sixth aspect of the present disclosure in preparation of a reagent for delivering a transgene to a cell.

In some embodiments, the cell is derived from a subject.

In some preferred embodiments, the subject is a mammalian subject.

In some more preferred embodiments, the subject is a human.

In some embodiments, the cell is derived from an eye.

In some embodiments, the subject suffers from an eye disease.

In some embodiments, the adeno-associated virus, the transgene delivery vector, or the pharmaceutical composition is administered to a subject via subretinal or intravitreal injection.

According to the eighth aspect of the present disclosure, there is provided use of the adeno-associated virus according to the fourth aspect of the present disclosure, the transgene delivery vector according to the fifth aspect of the present disclosure, or the pharmaceutical composition according to the sixth aspect of the present disclosure in preparation of a medicament for treating a disease.

In some embodiments, the disease comprises an eye disease.

According to the ninth aspect of the present disclosure, there is provided a method for treating a disease, comprising a step of administering, to a subject, the adeno-associated virus according to the fourth aspect of the present disclosure, the transgene delivery vector according to the fifth aspect of the present disclosure, or the pharmaceutical composition according to the sixth aspect of the present disclosure.

In some embodiments, the disease comprises an eye disease.

### Advantageous Effects of the Invention

The adeno-associated virus capsid protein and the adeno-associated virus comprising the same provided in the present disclosure have improved *in vivo* and/or *in vitro* cellular infection efficiencies as compared to the wild-type adeno-associated virus, have a good targeting capability to eyes, and can serve as a delivery vector for treatment of eye-associated diseases.

### BRIEF DESCRIPTION OF THE DRAWINGS

FIG. 1 is a schematic diagram showing the insertion site in a candidate AAV capsid protein and the plasmid packaging system for novel AAV capsids.
FIG. 2 shows the quantitative results of 15 candidate AAVs infecting human retinal epitheliums (ARPE-19), with the percentage of GFP-positive ARPE-19 cells as the statistical indicator.
FIG. 3 shows the transduction results of 4 candidate AAVs (AAV IVT8/IVT13/IVT14/IVT15) and the wild-type AAV2 into mouse retinae.
A in FIG. 4 shows the transduction results of 3 candidate AAVs (AAV IVT8/IVT13/IVT15) and the wild-type AAV2 after infecting human retinal organoids, and B in FIG. 4 shows the GFP proportion of retinal cells.
FIG. 5A shows the GFP fluorescence signals in retinas of mice intravitreally injected with AAV IVT13 and wild-type AAV2 (2E+9 vg/eye).
FIG. 5B shows intact retinal sections from mice transduced with AAV IVT13 and wild-type AAV2.
FIG. 5C shows retinal sections from mice transduced with AAV IVT13 and wild-type AAV2, showing the GFP, DAPI and the retinal ganglion marker RBPMS.
FIG. 5D shows retinal sections from mice transduced with AAV IVT13 and wild-type AAV2, showing the GFP, DAPI and the bipolar cell marker PKCα.
FIG. 5E shows retinal sections from mice transduced with AAV IVT13 and wild-type AAV2, showing the GFP, DAPI and the retinal pigment epithelium marker RPE65.
FIG. 6A shows retinal sections from monkeys transduced with AAV IVT13 and wild-type AAV2, showing the GFP, DAPI and the retinal bipolar cell marker PKCα.
FIG. 6B shows retinal sections from monkeys transduced with AAV IVT13 and wild-type AAV2, showing the GFP, DAPI and the retinal photosensitive layer marker RS1.
FIG. 7 shows the transduction efficiencies of AAV IVT13, AAV IVT13-P34A and wild-type AAV2 upon infecting ARPE-19 cells.
FIG. 8 shows the transduction results of AAV IVT13, AAV IVT13-P34A and wild-type AAV2 after infecting human retinal organoids.

### DETAILED DESCRIPTION

To render the present disclosure more understandable, some technical and scientific terms are specifically defined below. Unless otherwise explicitly defined herein, all of the other technical and scientific terms used herein have the same meanings as typically understood by one of ordinary skill in the art to which the present disclosure belongs.

In the present specification, the numerical range represented by "numerical value A to numerical value B" refers to the range including the endpoint values A and B.

In the present specification, the term "basically" or "substantially" means that the standard deviation from the theoretical model or theoretical data is within a range of 5%, preferably 3%, more preferably 1%.

In the present specification, the term "may" involves both the meaning of doing something and the meaning of not doing something.

In the present specification, the term "optional" or "optionally" means that the event or case described below may or may not occur, and this description includes the case where this event occurs and the case where this event does not occur.

In the present disclosure, the terms "comprising" and "having" and any variations thereof are intended to encompass non-exclusive inclusions. For example, a process, method, apparatus, product or device including a series of steps or modules is not confined to including the listed steps or modules, but may optionally include those unlisted, or may optionally include other steps or modules innate in the process, method, product or device.

The term "a plurality of" referred to in the present disclosure means two or more. The term "and/or" describes an association between the associated objects, and represents three possible relationships. For example, A and/or B may represent the following three cases: A exists alone, both A and B exist, and B exists alone. The character "/" typically denotes an alternative *(i.e.* "or") relationship between the associated objects it connects.

Phrases such as "some specific/preferred embodiments", "other specific/preferred embodiments", and "embodiments" referred to in the present specification mean that particular elements (for example, features, structures, properties and/or characteristics) described in relation to this embodiment are included in at least one of the embodiments described herein, and may or may not exist in other embodiments. Additionally, it should be appreciated that the elements may be combined in any suitable manner into various embodiments.

According to the present disclosure, "Vg" refers to Viral Genome and is equivalent to Genome Copy.

According to the present disclosure, the terms "polypeptide", "protein", and "peptide" are used interchangeably herein to refer to a polymeric form of amino acids of any length, which may include coded and non-coded amino acids, chemically or biochemically modified or derived amino acids, and polypeptides having similar peptide backbones.

According to the present disclosure, the terms "nucleic acid molecule", "polynucleotide", "polynucleic acid", and "nucleic acid" are used interchangeably to refer to a polymeric form of nucleotides of any length, regardless of deoxyribonucleotides or ribonucleotides, or analogs thereof. Polynucleotide may have any three-dimensional structure and may implement any known or unknown function. Non-limiting examples of the polynucleotide include genes, gene fragments, exons, introns, messenger RNA (mRNA), transfer RNA, ribosomal RNA, ribozymes, cDNA, recombinant polynucleotides, branched polynucleotides, plasmids, vectors, isolated DNA of any sequence, control regions, isolated RNA of any sequence, and nucleic acid probes and primers. The nucleic acid molecule may be linear or circular.

As used herein, the term "amino acid" may include natural amino acids, unnatural amino acids, amino acid analogs, and all of D and L stereoisomers thereof. According to the present disclosure, the three-letter and single-letter codes for amino acids used herein are as described in J. biol. chem, 243, p3558 (1968). In the present disclosure, the amino acids as well as their abbreviations and English codes are as follows: histidine (His, H); serine (Ser, S); glutamic acid (Glu, E); glutamine (Gln, Q); glycine (Gly, G); threonine (Thr, T); phenylalanine (Phe, F); aspartic acid (Asp, D); tyrosine (Tyr, Y); leucine (Leu, L); isoleucine (Ile, I); arginine (Arg, R); alanine (Ala, A); valine (Val, V); tryptophan (Trp, W); methionine (Met, M); asparagine (Asn, N); cysteine (Cys, C); lysine (Lys, K); proline (Pro, P).

According to the present disclosure, the "addition" of an amino acid means that an amino acid is added at the C-terminus or N-terminus of an amino acid sequence. According to the present disclosure, the "deletion" of an amino acid means that 1, 2 or 3 or more amino acids can be deleted from an amino acid sequence. According to the present disclosure, the "insertion" of an amino acid means that an amino acid residue is inserted at an appropriate position in an amino acid sequence, and the inserted amino acid residues may also be all or partially adjacent to one another, or the inserted amino acids are not adjacent to one another. According to the present disclosure, the "substitution" of an amino acid means that an amino acid residue at a position in an amino acid sequence is substituted with an additional amino acid residue, where the "substitution" may be a conservative substitution of an amino acid.

According to the present disclosure, the term "conservative modification", "conservative substitution" or "conservative replacement" refers to replacement of an amino acid in a protein with an additional amino acid having similar characteristics (e.g. charge, size of side chain, hydrophobicity/hydrophilicity, backbone conformation, and rigidity), such that the protein can be changed frequently without altering the biological activity of the protein. A person skilled in the art knows that in general, replacement of a single amino acid in the non-essential region of a polypeptide does not substantially alter the biological activity (see, for example, Watson et al. (1987) Molecular Biology of the Gene, The Benjamin/Cummings Pub. Co., page 224, (4th ed.)). Furthermore, replacement with structurally or functionally similar amino acids is unlikely to disrupt the biological activity. Exemplary conservative substitutions are set forth in the following "Exemplary Amino Acid Conservative Substitutions":

### Exemplary Amino Acid Conservative Substitutions

| Original Residue | Conservative Substitution |
|---|---|
| Ala(A) | Gly; Ser |
| Arg(R) | Lys; His |
| Asn(N) | Gln; His; Asp |
| Asp(D) | Glu; Asn |
| Cys(C) | Ser; Ala; Val |
| Gln(Q) | Asn; Glu |
| Glu(E) | Asp; Gln |
| Gly(G) | Ala |
| His(H) | Asn; Gln |
| Ile(I) | Leu; Val |
| Leu(L) | Ile; Val |
| Lys(K) | Arg; His |
| Met(M) | Leu; Ile; Tyr |
| Phe(F) | Tyr; Met; Leu |
| Pro(P) | Ala |
| Ser(S) | Thr |
| Thr(T) | Ser |
| Trp(W) | Tyr; Phe |
| Tyr(Y) | Trp; Phe |
| Val(V) | Ile; Leu |

According to the present disclosure, "moderate to very high stringency conditions" include "moderate stringency conditions", "moderate-high stringency conditions", "high stringency conditions" or "very high stringency conditions", which describe the conditions for hybridization and washing of nucleic acids. Guidance for conducting hybridization reactions can be found in Current Protocols in Molecular Biology, John Wiley & Sons, N.Y. (1989), 6.3.1-6.3.6, which is incorporated herein by reference. This literature describes both aqueous and non-aqueous processes and either can be used. For example, specific hybridization conditions are as follows: (1) the low stringency hybridization conditions refer to hybridizing in 6× sodium chloride/sodium citrate (SSC) at about 45°C, and then washing twice in 0.2× SSC and 0.1% SDS at 50°C or higher (the washing temperature can be raised to 55°C in the case of the low stringency conditions); (2) the moderate stringency hybridization conditions refer to hybridizing in 6× SSC at about 45°C, and then washing one or more times in 0.2× SSC and 0.1% SDS at 60°C; (3) the high stringency hybridization conditions refer to hybridizing in 6× SSC at about 45°C, and then washing one or more times in 0.2× SSC and 0.1% SDS at 65°C, which is preferred; and (4) the very high stringency hybridization conditions refer to hybridizing in 0.5 M sodium phosphate and 7% SDS at 65°C, and then washing one or more times in 0.2× SSC and 1% SDS at 65°C.

The term "identity" refers to the sequence similarity between two polynucleotide sequences or between two polypeptides. When the positions in two compared sequences are all occupied by the same base or amino acid monomer subunit, for example, if each of the positions in two DNA molecules is occupied by adenine, the molecules are homologous at this position. The percent identity between two sequences is a function of the number of matching or homologous positions shared by the two sequences / the number of positions to be compared × 100%. For example, in the case of optimal alignment of sequences, if six out of ten positions in two sequences are matched or homologous, the two sequences are 60% homologous. In general, comparison is made between two sequences when aligned to obtain the maximum percent identity.

When applied to an animal, human, test subject, cell, tissue, organ or biological fluid, the terms "administration", "giving", and "treating" refer to contact of an exogenous drug, therapeutic agent, diagnostic agent, or composition with the animal, human, subject, cell, tissue, organ or biological fluid. The terms "administration", "giving", and "treating" may refer to, e.g., therapeutic, pharmacokinetics, diagnostic, research, and experimental methods. Treating of cells includes contact of a reagent with the cells and contact of a reagent with a fluid, where the fluid is in contact with the cells. The terms "administration", "giving", and "treating" also means *in vitro* and *ex vivo* treatment of, for example, a cell, by a reagent, a diagnostic agent, a binding composition or an additional cell. When applied to humans, veterinary, or research subjects, "treating" refers to therapeutic, prophylactic or precautionary measures, research and diagnostic applications.

The term "treatment" means administering a therapeutic agent for internal or external use, such as a therapeutic agent comprising any of the antibodies described herein, to a subject having one or more symptoms of a disease, on which the therapeutic agent is known to have a therapeutic effect. Generally, the therapeutic agent is administered in an amount effective to alleviate one or more symptoms of the disease in the subject or population being treated by either inducing regression of such symptoms or suppressing progression of such symptoms to any clinically measurable degree. The amount of a therapeutic agent effective to alleviate any specific disease symptom (also referred to as a "therapeutically effective amount") may vary depending upon multiple factors such as the disease state, age, and body weight of the patient, and the ability of the drug to produce the desired therapeutic effect in the patient. Any clinical test method typically used by a physician or other health care professional to assess the severity or progression of a disease symptom may be adopted to evaluate whether this symptom has been alleviated.

In the present specification, the term "prevention" refers to prophylactic treatment on a subject who does not have a disease now or in the past but is at risk of developing a disease or who had a disease in the past and does not have a disease now but is at risk of recurrence of the disease. In some embodiments, the subject runs a higher risk of developing a disease or a higher risk of recurrence of the disease as compared to the members at the average healthy level in the subject population.

An "effective amount" includes an amount sufficient to ameliorate or prevent the symptoms of a medical condition or the condition. An effective amount also means an amount sufficient to allow for or facilitate diagnosis. An effective amount for a particular patient or veterinary subject may vary depending on factors such as the condition to be treated, the patient's overall health, the method, route, and dose of administration, and the severity of side effects. An effective amount may be the maximum dose or dosage regimen that avoids significant side effects or toxic effects.

In the present specification, the "therapeutically effective amount" is an amount sufficient to provide a therapeutic benefit in the course of treating a condition or sufficient to delay or minimize one or more symptoms associated with the condition. A therapeutically effective amount refers to an amount of a therapeutic agent alone or in combination with other therapies, which provides therapeutic benefits in the course of treating a condition. The term "therapeutically effective amount" may include an amount that improves overall therapy; reduces or avoids symptoms, signs, or causes of a condition; and/or enhances the therapeutic efficacy of an additional therapeutic agent.

In the present specification, the "prophylactically effective amount" is an amount sufficient to prevent a condition or one or more symptoms associated with the condition or to prevent its recurrence. A prophylactically effective amount refers to an amount of a therapeutic agent alone or in combination with other drugs, which provides therapeutic benefits in the course of preventing a condition. The term "prophylactically effective amount" may include an amount that improves overall prevention or enhances the prophylactic efficacy of an additional prophylactic agent.

In the present specification, the term "subject" refers to a human *(i.e.,* a male or female of any age, *e.g.,* a pediatric subject *(e.g.,* an infant, child, or adolescent) or an adult subject *(e.g.,* a young, middle-aged, or elderly person)) or a non-human animal. In some embodiments, the non-human animal is a mammal *(e.g.,* a primate (such as *Macaca fascicularis* or *Macaca mulatta*), a commercially relevant mammal (such as cow, pig, horse, sheep, goat, cat, or dog), or a bird. The non-human animal may be male or female at any stage of development. The non-human animal may be a transgenic animal or a genetically engineered animal.

### Detailed Description of the Invention

### <AAV Capsid Protein and Targeting Peptide>

In some embodiments of the present disclosure, there is provided an adeno-associated virus (AAV) capsid protein, comprising a targeting peptide, wherein the targeting peptide comprises one or more of the following sequences:
(i) a sequence AAX₁X₂X₃X₄X₅X₆X₇X₈AA; and
(ii) a sequence NG.

In some specific embodiments of the present disclosure, in the sequence AAX₁X₂X₃X₄X₅X₆X₇X₈AA:
(a) X₁ is A;
(b) X₂ is R or G;
(c) X₃ is G or N;
(d) X₄ is D, S or G;
(e) X₅ is L or R;
(f) X₆ is A, G, Q or a deletion;
(g) X₇ is T, R, N, A, H or a deletion; and
(h) X₈ is I, L, P, A or a deletion.

Exemplarily, the targeting peptide comprises an amino acid sequence as set forth in any one of SEQ ID NOs: 1 to 14.

In some more specific embodiments of the present disclosure, in the sequence AAX₁X₂X₃X₄X₅X₆X₇X₈AA:
(a) X₁ is A;
(b) X₂ is R;
(c) X₃ is G;
(d) X₄ is D or S;
(e) X₅ is L;
(f) X₆ is A, G, Q or a deletion;
(g) X₇ is T, R, N, A or a deletion; and
(h) X₈ is I, L, P or a deletion.

Exemplarily, the targeting peptide comprises an amino acid sequence as set forth in any one of SEQ ID NOs: 1 to 13.

In some preferred embodiments of the present disclosure, in the sequence AAX₁X₂X₃X₄X₅X₆X₇X₈AA:
(a) X₁ is A;
(b) X₂ is R;
(c) X₃ is G;
(d) X₄ is D or S;
(e) X₅ is L;
(f) X₆ is G, Q or a deletion;
(g) X₇ is R, N or a deletion; and
(h) X₈ is P, I, L or a deletion.

Exemplarily, the targeting peptide comprises an amino acid sequence as set forth in any one of SEQ ID NOs: 2, 8, 11, 12, and 13.

In some preferred embodiments of the present disclosure, in the sequence AAX₁X₂X₃X₄X₅X₆X₇X₈AA:
(a) X₁ is A;
(b) X₂ is R;
(c) X₃ is G;
(d) X₄ is D;
(e) X₅ is L;
(f) X₆ is G;
(g) X₇ is R; and
(h) X₈ is L or P.

Exemplarily, the targeting peptide comprises an amino acid sequence as set forth in any one of SEQ ID NOs: 2 and 8.

In other preferred embodiments of the present disclosure, in the sequence AAX₁X₂X₃X₄X₅X₆X₇X₈AA:
(a) X₁ is A;
(b) X₂ is R;
(c) X₃ is G;
(d) X₄ is S;
(e) X₅ is L;
(f) X₆ is Q or a deletion;
(g) X₇ is N or a deletion; and
(h) X₈ is I, L or a deletion.

Exemplarily, the targeting peptide comprises an amino acid sequence as set forth in any one of SEQ ID NOs: 11, 12, and 13.

In other preferred embodiments of the present disclosure, the targeting peptide comprises the amino acid sequence: SEQ ID NO: 14.

In some preferred embodiments of the present disclosure, the targeting peptide comprises an amino acid sequence as set forth in any one of SEQ ID Nos: 1 to 14 and the sequence NG.

In some more preferred embodiments of the present disclosure, the targeting peptide comprises an amino acid sequence as set forth in any one of SEQ ID NOs: 2, 8, 11, 12, 13, and 14 and the sequence NG.

In some further preferred embodiments of the present disclosure, the targeting peptide comprises an amino acid sequence as set forth in any one of SEQ ID NOs: 8, 13, and 14 and the sequence NG.

In some further preferred embodiments of the present disclosure, the targeting peptide comprises an amino acid sequence as set forth in any one of SEQ ID NOs: 8, 13 and 14.

In the present disclosure, AAV is a tiny non-enveloped virus having a 25 nm capsid. No disease is known or has been shown to be associated with the wild type virus. AAV has a single-stranded DNA (ssDNA) genome. AAV has been shown to exhibit long-term episomal transgene expression. Vectors containing as few as 300 base pairs of AAV can be packaged and can integrate. Space for exogenous DNA is limited to about 4.7 kb. An AAV vector such as that described in Tratschin et al., Mol. Cell. Biol. 5:3251-3260 (1985) can be used to introduce DNA into cells. A variety of nucleic acids have been introduced into different cell types using AAV vectors (see for example Hermonat et al., Proc. Natl. Acad. Sci. USA 81:6466-6470 (1984); Tratschin et al., Mol. Cell. Biol. 4:2072-2081 (1985); Wondisford et al., Mol. Endocrinol. 2:32-39 (1988); Tratschin et al., J. Virol. 51:611-619 (1984); and Flotte et al., J. Biol. Chem. 268:3781-3790 (1993)). There are numerous alternative AAV variants (over 100 have been cloned), and AAV variants have been identified based on desirable characteristics.

In some embodiments, AAV is one or more selected from AAV1, AAV2, AAV3, AAV4, AAV5, AAV6, AAV7, AAV8, AAV9, rh.10, rh.39, rh.43, and rh.74.

In some preferred embodiments, AAV is AAV2.

AAV capsid is an icosahedron composed of 60 VP capsid protein monomers including 5 VP1 monomers, 5 VP2 monomers, and 50 VP3 monomers. VP1, VP2, and VP3 monomers are all transcribed and translated by the *Cap* gene of AAV. VP1 is the longest and includes approximate 735 amino acids. VP2 and VP3 are "truncated forms" of VP1 and do not contain part of amino acids at the N-terminus of the VP1 protein. Conventionally, the modification sites in the capsid protein are designated based on the amino acid sequence of the VP1 protein. For instance, insertion of a targeting peptide after the 587^{th} amino acid in the capsid/capsid protein of AAV2 means that the targeting peptide is inserted after the 587^{th} amino acid of the VP1 monomer (containing 735 amino acids) in AAV2, so that all of AAV2 VP1, VP2 and VP3 contain this targeting peptide at the corresponding position. The amino acid sequence of the capsid/capsid protein in AAV2 is typically represented by the amino acid sequence of VP1.

In some embodiments, AAV2 includes AAV2 VP1 or a variant thereof, AAV2 VP2 or a variant thereof and/or AAV2 VP3 or a variant thereof.

Exemplarily, the amino acid sequence of AAV2 VP1 is as shown below (SEQ ID NO: 60): 












*
 (a total of 735 amino acids, the insertion site of the targeting peptide is between amino acids N587 and R588)

Exemplarily, the variant of AAV2 VP1 includes a variant in which proline (P) at position 34 is mutated to alanine (A) relative to the amino acid sequence as set forth in SEQ ID NO: 60.

The amino acid sequence of AAV2 VP2 is as shown below (SEQ ID NO: 61): 
MAPGKKRPVEHSPVEPDSSSGTGKAGQQPARKRLNFGQTGDADSVPDPQPLGQPP
AAPSGLGTNTMATGSGAPMADNNEGADGVGNSSGNWHCDSTWMGDRVITTSTRTWA
LPTYNNHLYKQISSQSGASNDNHYFGYSTPWGYFDFNRFHCHFSPRDWQRLINNNWG
FRPKRLNFKLFNIQVKEVTQNDGTTTIANNLTSTVQVFTDSEYQLPYVLGSAHQGCLPP
FPADVFMVPQYGYLTLNNGSQAVGRSSFYCLEYFPSQMLRTGNNFTFSYTFEDVPFHSS
 
YAHSQSLDRLMNPLIDQYLYYLSRTNTPSGTTTQSRLQFSQAGASDIRDQSRNWLPGPC
YRQQRVSKTSADNNNSEYSWTGATKYHLNGRDSLVNPGPAMASHKDDEEKFFPQSGV
LIFGKQGSEKTNVDIEKVMITDEEEIRTTNPVATEQYGSVSTNLQRGNRQAATADVNTQ
GVLPGMVWQDRDVYLQGPIWAKIPHTDGHFHPSPLMGGFGLKHPPPQILIKNTPVPAN
PSTTFSAAKFASFITQYSTGQVSVEIEWELQKENSKRWNPEIQYTSNYNKSVNVDFTVD
TNGVYSEPRPIGTRYLTRNL*
 (a total of 598 amino acids, corresponding to the amino acids 138-736 in AAV2 VP1, the insertion site of the targeting peptide is between amino acids N450 and R451, namely, corresponding to between amino acids N587 and R588 in AAV2 VP1)

The amino acid sequence of AAV2 VP3 is as shown below (SEQ ID NO: 62): MATGSGAPMADNNEGADGVGNSSGNWHCDSTWMGDRVITTSTRTWALPTY NNHLYKQISSQSGASNDNHYFGYSTPWGYFDFNRFHCHFSPRDWQRLINNNWGFRPK RLNFKLFNIQVKEVTQNDGTTTIANNLTSTVQVFTDSEYQLPYVLGSAHQGCLPPFPAD VFMVPQYGYLTLNNGSQAVGRSSFYCLEYFPSQMLRTGNNFTFSYTFEDVPFHSSYAH SQSLDRLMNPLIDQYLYYLSRTNTPSGTTTQSRLQFSQAGASDIRDQSRNWLPGPCYRQ QRVSKTSADNNNSEYSWTGATKYHLNGRDSLVNPGPAMASHKDDEEKFFPQSGVLIFG KQGSEKTNVDIEKVMITDEEEIRTTNPVATEQYGSVSTNLQRGNRQAATADVNTQGVL PGMVWQDRDVYLQGPIWAKIPHTDGHFHPSPLMGGFGLKHPPPQILIKNTPVPANPSTT FSAAKFASFITQYSTGQVSVEIEWELQKENSKRWNPEIQYTSNYNKSVNVDFTVDTNG VYSEPRPIGTRYLTRNL* (a total of 533 amino acids, corresponding to the amino acids 203-736 in AAV2 VP1, the insertion site of the targeting peptide is between the amino acids N385 and R386, namely, corresponding to between amino acids N587 and R588 in AAV2 VP1)

In some embodiments of the present disclosure, the targeting peptide is inserted at a position corresponding to the 587^{th} amino acid in AAV2 VP1 or a variant thereof. That is, the targeting peptide is located between the 587^{th} amino acid and the 588^{th} amino acid in AAV2 VP1 or a variant thereof. In some specific embodiments of the present disclosure, when the targeting peptide is inserted at a position corresponding to the 587^{th} amino acid in AAV2 VP1 or a variant thereof, the targeting peptide comprises the sequence AAX₁X₂X₃X₄X₅X₆X₇X₈AA.

In some embodiments of the present disclosure, the targeting peptide substitutes for the 586^{th} amino acid and the 587^{th} amino acid in AAV2 VP1 or a variant thereof. In some specific embodiments of the present disclosure, when the targeting peptide substitutes for the 586^{th} amino acid and the 587^{th} amino acid in AAV2 VP1 or a variant thereof, the targeting peptide comprises the sequence NG.

In some preferred embodiments of the present disclosure, the AAV capsid protein comprises an amino acid sequence as set forth in any one of SEQ ID NOs: 15 to 29 and 65.

In some specific embodiments, the sequence of AAV may have, *e.g.,* at least 80, 85, 90, 95, 97 or 99% identity to the exemplary AAV sequence described herein, for example, it is possible to include a variant, preferably the one that does not reduce the ability of AAV to mediate the transgene expression in cells.

### <Polynucleotide>

In some embodiments of the present disclosure, there is provided a polynucleotide encoding the AAV capsid protein according to the present disclosure or encoding the targeting peptide according to the present disclosure.

The polynucleotide of the present disclosure may be in a DNA or RNA form. The DNA form includes cDNA, genomic DNA or synthetic DNA. DNA may be single-stranded or double-stranded. DNA may be a coding strand or a non-coding strand.

The polynucleotide encoding the AAV capsid protein according to the present disclosure includes: a coding sequence encoding only the AAV capsid protein/targeting peptide; a coding sequence of the AAV capsid protein/targeting peptide and various additional coding sequences; and a coding sequence (and optionally additional coding sequences) of the AAV capsid protein/targeting peptide and a non-coding sequence.

The "polynucleotide encoding the AAV capsid protein/targeting peptide" may be a polynucleotide containing the coding sequence encoding the AAV capsid protein/targeting peptide, or may be a polynucleotide further containing an additional coding sequence and/or a non-coding sequence.

The present disclosure further relates to a polynucleotide whose sequence hybridizes with and has at least 50%, preferably at least 70%, more preferably at least 80% identity to the above-mentioned sequence. The present disclosure relates particularly to a polynucleotide hybridizable with the polynucleotide according to the present disclosure under stringency conditions. The stringency conditions are moderate stringency conditions, moderate-high stringency conditions, high stringency conditions or very high stringency conditions.

### <AAV and Transgene Delivery Vector>

In some embodiments of the present disclosure, there is provided an AAV, comprising the AAV capsid protein according to the present disclosure.

According to some aspects of the present disclosure, there is further provided a transgene delivery vector, comprising the AAV according to the present disclosure.

The AAV and transgene delivery vector according to the present disclosure can be used to deliver any composition (e.g., a sequence of interest) to the tissue, for example, retina.

In some embodiments, the AAV further comprises a transgene.

In some specific embodiments, the transgene comprises a nucleotide sequence encoding a gene product. "Gene" refers to a polynucleotide containing at least one open reading frame capable of encoding a specific protein after transcription and translation. "Gene product" is a molecule resulting from expression of a particular gene. The gene product includes, e.g., polypeptides, aptamers, interfering RNAs, and mRNAs.

In some specific embodiments, the transgene is a therapeutic transgene, a prophylactic transgene, or a diagnostic transgene.

In some exemplary embodiments, the transgene is a therapeutic transgene encoding the gene sequence of a therapeutic agent, *e.g.,* encoding the gene sequence of treating eye diseases.

In other exemplary embodiments, the transgene is a gene sequence encoding a reporter protein, such as a fluorescent protein (an enzyme that produces a detectable product by catalysis).

### <Pharmaceutical Composition and Methods of Administration>

In some embodiments of the present disclosure, there is provided a pharmaceutical composition, comprising:
(a) the AAV or the transgene delivery vector as described above; and optionally
(b) a pharmaceutically acceptable carrier.

In some embodiments, the pharmaceutical composition according to the present disclosure contains the aforementioned AAV or the aforementioned transgene delivery vector as an active ingredient, the AAV or the transgene delivery vector comprising (i) a targeting peptide and (ii) a transgene, for example, a therapeutic transgene, exemplarily, therapeutic agents for treating eye diseases.

As used herein, the term "pharmaceutically acceptable carrier" includes saline, solvents, dispersion media, coatings, antibacterial and antifungal agents, isotonic and absorption delaying agents, and the like, compatible with pharmaceutical administration.

Pharmaceutical compositions are typically formulated to be compatible with its intended route of administration. Examples of routes of administration include parenteral, e.g., intravenous, intraarterial, subcutaneous, intraperitoneal, intrathecal, and intramuscular administration via either injection or infusion. Delivery can thus be systemic or localized. For example, in order to deliver to the retina, subretinal or intravitreal injection may be taken (see for example Ochakovski et al., Front Neurosci. 2017; 11: 174; Xue et al., Eye (Lond). 2017 Sep; 31(9):1308-1316).

Methods of formulating suitable pharmaceutical compositions are known in the art, see, *e.g.,* Remington: The Science and Practice of Pharmacy, 21st ed., 2005; and the books in the series *Drugs and the Pharmaceutical Sciences: a Series of Textbooks and Monographs* (Dekker, NY).

### <Uses and Methods>

In some embodiments of the present disclosure, there is provided use of the AAV according to the present disclosure, the transgene delivery vector according to the present disclosure and/or the pharmaceutical composition according to the present disclosure in preparation of a reagent for delivering a transgene to a cell.

In other embodiments of the present disclosure, there is provided a method for delivering a transgene to a cell, the method comprising a step of bringing the cell into contact with the AAV according to the present disclosure, the transgene delivery vector according to the present disclosure and/or the pharmaceutical composition according to the present disclosure.

The AAV according to the present disclosure can be used to deliver a transgene such as a therapeutic transgene to a tissue, *e.g.,* to the eyes of the subject. In some embodiments, the AAV according to the present disclosure can deliver the transgene/therapeutic transgene to retinal cells, exemplarily, retinal photoreceptors, interneurons, retinal ganglion cells, or retinal pigment epitheliums (RPEs).

In some embodiments, the AAVs according to the present disclosure are used to deliver a nucleic acid sequence encoding a therapeutic agent to a subject who has an eye disease.

In some specific embodiments of the present disclosure, there is provided use of the AAV according to the present disclosure, the transgene delivery vector according to the present disclosure and/or the pharmaceutical composition according to the present disclosure in preparation of a medicament for treating a disease.

In other specific embodiments of the present disclosure, there is provided a method for treating a disease, comprising a step of administering, to a subject, the AAV according to the present disclosure, the transgene delivery vector according to the present disclosure and/or the pharmaceutical composition according to the present disclosure.

In the present disclosure, the disease may be any disease that can be treated by an AAV carrying a transgene. In some preferred embodiments, the disease may be an eye disease. In some specific embodiments, the eye disease includes, but is not limited to, neovascular Wet Age-related Macular Degeneration (wetAMD), Bietti crystalline corneoretinal dystrophy (BCD), X-linked juvenile retinoschisis (XLRS), Leber's Hereditary Optic Neuropathy (LHON), Leber's congenital amaurosis, (LCA) and the like.

### Examples

The present disclosure will be described below in greater details with reference to the specific embodiments. The examples described below are merely intended to illustrate the present disclosure, rather than to limit its scope. The examples provided below may serve as guidance for further improvements by one of ordinary skill in the art and are not intended to limit the present disclosure in any way.

Unless otherwise specified, the experimental methods in the following examples are all conventional methods and are carried out according to the techniques or conditions described in the literatures in this field or according to the product instructions. All the materials, reagents and the like used in the following examples are commercially available, unless otherwise specified.

### Materials and Methods

HEK 293T cell line (ATCC, CRL-3216);
Human retinal epitheliums (ARPE-19, Pricella, Cat. No.: CL-0026);
Fetal bovine serum (Gibco, A3161002C);
Dulbecco's Modified Eagle Medium (DMEM medium, Gibco, 11965092);
Dulbecco's Modified Eagle Medium, F-12 (DMEM/F12 medium, Gibco, 11320-033)
Penicillin-Streptomycin (Gibco, 15140122);
0.05% Trypsin-EDTA (Gibco, 25300062);
Opti-MEM (Gibco, 31985070);
Poly ethylenimine Linear (PEI) MW40000 (YEASEN, 40816ES03);
D-PBS containing Ca²⁺, Mg²⁺ (Corning, 21-030-CV);
24-well TC-treated culture plate (Costar, 3524);
100 mm TC-treated culture dish (Jet Bio-Filtration, TCD010100);
15 mL Centrifuge Tube (Axygen, SCT-15ML-25-S);
Serological Pipets (Jet Bio-Filtration);
Benzonase (Sigma-Aldrich, E8263-25KU);
Q5^{®} High-Fidelity 2X Master Mix (NEB, M0492L).

### Example 1: Analysis and Design of Targets

In this example, suitable peptides were designed to broadly target retinal cells. This example designed 15 potential targeting peptides (Table 1 below).

**Table 1:**

| Targeting peptide | SEQ ID NO: | Sequence |
|---|---|---|
| Targeting peptide 1 | 1 | AAARGDLATIAA |
| Targeting peptide 2 | 2 | AAARGDLGRLAA |
| Targeting peptide 3 | 3 | AAARGDLQNIAA |
| Targeting peptide 4 | 4 | AAARGDLQNLAA |
| Targeting peptide 5 | 5 | AAARGDLAAIAA |
| Targeting peptide 6 | 6 | AAARGDLATLAA |
| Targeting peptide 7 | 7 | AAARGDLATPAA |
| Targeting peptide 8 | 8 | AAARGDLGRPAA |
| Targeting peptide 9 | 9 | AAARGDLGTLAA |
| Targeting peptide 10 | 10 | AAARGDLARIAA |
| Targeting peptide 11 | 11 | AAARGSLQNIAA |
| Targeting peptide 12 | 12 | AAARGSLQNLAA |
| Targeting peptide 13 | 13 | AAARGSLAA |
| Targeting peptide 14 | / | NG |
| Targeting peptide 15 | 14 | AAAGNGRAHAAA |

### Example 2: Design and Construction of AAV Capsid Protein Expression Plasmid Encoding Capsid Protein of Interest

### 1. Construction of RC2_IVB-NotI plasmid as intermediate plasmid

In this example, a reverse P5 promoter sequence was added upstream of the Rep sequence in pAAV-RC2 (purchased from CellBiolabs, Cat. No.: VPK-410-SER2), and a NotI restriction endonuclease site was inserted at 1752bp of the Cap2 sequence. General Biosystems (Anhui) Corp. Ltd. was commissioned to construct this intermediate plasmid RC2_IVB-NotI. The specific sequence was as shown below:

RC2_IVB-NotI plasmid sequence (SEQ ID NO: 63)
GCGCGCCGATATCTTCAAACCTCCCGCTTCAAAATGGAGACCCTGCGTGCTCAC
TCGGGCCCCCCCCCCCAGCGTGACCACATGGTGTCGCAAAATGTCGCAAAACACTC
ACGTGACCTCTAATACAGGACCTCCCTAACCCTATGACGTAATTCACGTCACGACTC
CACCCCTCCGTTAACGCCCCGCGCCGGCCGCTCTAGAACTAGTGGATCCCCCGGAA
GATCAGAAGTTCCTATTCCGAAGTTCCTATTCTCTAGAAAGTATAGGAACTTCTGATC
TGCGCAGCCGCCATGCCGGGGTTTTACGAGATTGTGATTAAGGTCCCCAGCGACCTT
GACGAGCATCTGCCCGGCATTTCTGACAGCTTTGTGAACTGGGTGGCCGAGAAGGA
ATGGGAGTTGCCGCCAGATTCTGACATGGATCTGAATCTGATTGAGCAGGCACCCCT
GACCGTGGCCGAGAAGCTGCAGCGCGACTTTCTGACGGAATGGCGCCGTGTGAGT
AAGGCCCCGGAGGCCCTTTTCTTTGTGCAATTTGAGAAGGGAGAGAGCTACTTCCA
CATGCACGTGCTCGTGGAAACCACCGGGGTGAAATCCATGGTTTTGGGACGTTTCC

TGAGTCAGATTCGCGAAAAACTGATTCAGAGAATTTACCGCGGGATCGAGCCGACT
TTGCCAAACTGGTTCGCGGTCACAAAGACCAGAAATGGCGCCGGAGGCGGGAACA
AGGTGGTGGATGAGTGCTACATCCCCAATTACTTGCTCCCCAAAACCCAGCCTGAG
CTCCAGTGGGCGTGGACTAATATGGAACAGTATTTAAGCGCCTGTTTGAATCTCACG
GAGCGTAAACGGTTGGTGGCGCAGCATCTGACGCACGTGTCGCAGACGCAGGAGC
AGAACAAAGAGAATCAGAATCCCAATTCTGATGCGCCGGTGATCAGATCAAAAACT
TCAGCCAGGTACATGGAGCTGGTCGGGTGGCTCGTGGACAAGGGGATTACCTCGGA
GAAGCAGTGGATCCAGGAGGACCAGGCCTCATACATCTCCTTCAATGCGGCCTCCA
ACTCGCGGTCCCAAATCAAGGCTGCCTTGGACAATGCGGGAAAGATTATGAGCCTG
ACTAAAACCGCCCCCGACTACCTGGTGGGCCAGCAGCCCGTGGAGGACATTTCCAG
CAATCGGATTTATAAAATTTTGGAACTAAACGGGTACGATCCCCAATATGCGGCTTCC
GTCTTTCTGGGATGGGCCACGAAAAAGTTCGGCAAGAGGAACACCATCTGGCTGTT
TGGGCCTGCAACTACCGGGAAGACCAACATCGCGGAGGCCATAGCCCACACTGTGC
CCTTCTACGGGTGCGTAAACTGGACCAATGAGAACTTTCCCTTCAACGACTGTGTC
GACAAGATGGTGATCTGGTGGGAGGAGGGGAAGATGACCGCCAAGGTCGTGGAGT
CGGCCAAAGCCATTCTCGGAGGAAGCAAGGTGCGCGTGGACCAGAAATGCAAGTC
CTCGGCCCAGATAGACCCGACTCCCGTGATCGTCACCTCCAACACCAACATGTGCG
CCGTGATTGACGGGAACTCAACGACCTTCGAACACCAGCAGCCGTTGCAAGACCG
GATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGA
GCATGAATTCTACGTCAAAAAGGGTGGAGCCAAGAAAAGACCCGCCCCCAGTGAC
GCAGATATAAGTGAGCCCAAACGGGTGCGCGAGTCAGTTGCGCAGCCATCGACGTC
AGACGCGGAAGCTTCGATCAACTACGCAGACAGGTACCAAAACAAATGTTCTCGTC
ACGTGGGCATGAATCTGATGCTGTTTCCCTGCAGACAATGCGAGAGAATGAATCAG
AATTCAAATATCTGCTTCACTCACGGACAGAAAGACTGTTTAGAGTGCTTTCCCGTG
TCAGAATCTCAACCCGTTTCTGTCGTCAAAAAGGCGTATCAGAAACTGTGCTACATT
CATCATATCATGGGAAAGGTGCCAGACGCTTGCACTGCCTGCGATCTGGTCAATGTG
GATTTGGATGACTGCATCTTTGAACAATAAATGATTTAAATCAGGTATGGCTGCCGAT
GGTTATCTTCCAGATTGGCTCGAGGACACTCTCTCTGAAGGAATAAGACAGTGGTG
GAAGCTCAAACCTGGCCCACCACCACCAAAGCCCGCAGAGCGGCATAAGGACGAC
AGCAGGGGTCTTGTGCTTCCTGGGTACAAGTACCTCGGACCCTTCAACGGACTCGA
CAAGGGAGAGCCGGTCAACGAGGCAGACGCCGCGGCCCTCGAGCACGACAAAGC
CTACGACCGGCAGCTCGACAGCGGAGACAACCCGTACCTCAAGTACAACCACGCC
GACGCGGAGTTTCAGGAGCGCCTTAAAGAAGATACGTCTTTTGGGGGCAACCTCGG
ACGAGCAGTCTTCCAGGCGAAAAAGAGGGTTCTTGAACCTCTGGGCCTGGTTGAG

GAACCTGTTAAGACGGCTCCGGGAAAAAAGAGGCCGGTAGAGCACTCTCCTGTGG
AGCCAGACTCCTCCTCGGGAACCGGAAAGGCGGGCCAGCAGCCTGCAAGAAAAA
GATTGAATTTTGGTCAGACTGGAGACGCAGACTCAGTACCTGACCCCCAGCCTCTC
GGACAGCCACCAGCAGCCCCCTCTGGTCTGGGAACTAATACGATGGCTACAGGCAG
TGGCGCACCAATGGCAGACAATAACGAGGGCGCCGACGGAGTGGGTAATTCCTCG
GGAAATTGGCATTGCGATTCCACATGGATGGGCGACAGAGTCATCACCACCAGCAC
CCGAACCTGGGCCCTGCCCACCTACAACAACCACCTCTACAAACAAATTTCCAGCC
AATCAGGAGCCTCGAACGACAATCACTACTTTGGCTACAGCACCCCTTGGGGGTATT
TTGACTTCAACAGATTCCACTGCCACTTTTCACCACGTGACTGGCAAAGACTCATC
AACAACAACTGGGGATTCCGACCCAAGAGACTCAACTTCAAGCTCTTTAACATTCA
AGTCAAAGAGGTCACGCAGAATGACGGTACGACGATTGCCAATAACCTTACCA
GCACGGTTCAGGTGTTTACTGACTCGGAGTACCAGCTCCCGTACGTCCTCGGCTCG
GCGCATCAAGGATGCCTCCCGCCGTTCCCAGCAGACGTCTTCATGGTGCCACAGTAT
GGATACCTCACCCTGAACAACGGGAGTCAGGCAGTAGGACGCTCTTCATTTTACTG
CCTGGAGTACTTTCCTTCTCAGATGCTGCGTACCGGAAACAACTTTACCTTCAGCTA
CACTTTTGAGGACGTTCCTTTCCACAGCAGCTACGCTCACAGCCAGAGTCTGGACC
GTCTCATGAATCCTCTCATCGACCAGTACCTGTATTACTTGAGCAGAACAAACACTC
CAAGTGGAACCACCACGCAGTCAAGGCTTCAGTTTTCTCAGGCCGGAGCGAGTGA
CATTCGGGACCAGTCTAGGAACTGGCTTCCTGGACCCTGTTACCGCCAGCAGCGAG
TATCAAAGACATCTGCGGATAACAACAACAGTGAATACTCGTGGACTGGAGCTACC
AAGTACCACCTCAATGGCAGAGACTCTCTGGTGAATCCGGGCCCGGCCATGGCAAG
CCACAAGGACGATGAAGAAAAGTTTTTTCCTCAGAGCGGGGTTCTCATCTTTGGGA
AGCAAGGCTCAGAGAAAACAAATGTGGACATTGAAAAGGTCATGATTACAGACGA
AGAGGAAATCAGGACAACCAATCCCGTGGCTACGGAGCAGTATGGTTCTGTATCTA
CCAACCTCCAGCGCGGCCGCAACAGACAAGCAGCTACCGCAGATGTCAACACACA
AGGCGTTCTTCCAGGCATGGTCTGGCAGGACAGAGATGTGTACCTTCAGGGGCCCA
TCTGGGCAAAGATTCCACACACGGACGGACATTTTCACCCCTCTCCCCTCATGGGT
GGATTCGGACTTAAACACCCTCCTCCACAGATTCTCATCAAGAACACCCCGGTACCT
GCGAATCCTTCGACCACCTTCAGTGCGGCAAAGTTTGCTTCCTTCATCACACAGTAC
TCCACGGGACAGGTCAGCGTGGAGATCGAGTGGGAGCTGCAGAAGGAAAACAGC
AAACGCTGGAATCCCGAAATTCAGTACACTTCCAACTACAACAAGTCTGTTAATGTG
GACTTTACTGTGGACACTAATGGCGTGTATTCAGAGCCTCGCCCCATTGGCACCAGA
TACCTGACTCGTAATCTGTAATTGCTTGTTAATCAATAAACCGTTTAATTCGTTTCAG
TTGAACTTTGGTCTCTGCGTATTTCTTTCTTATCTAGTTTCCATGGCTACGTAGATAAG
TAGCATGGCGGGTTAATCATTAACTACAGCCCGGGCGTTTAAACAGCGGGCGGAGG

GGTGGAGTCGTGACGTGAATTACGTCATAGGGTTAGGGAGGTCCTGTATTAGAGGTC
ACGTGAGTGTTTTGCGACATTTTGCGACACCATGTGGTCTCGCTGGGGGGGGGGGC
CCGAGTGAGCACGCAGGGTCTCCATTTTGAAGCGGGAGGTTTGAACGAGCGCTGG
CGCGCTCACTGGCCGTCGTTTTACAACGTCGTGACTGGGAAAACCCTGGCGTTACC
CAACTTAATCGCCTTGCAGCACATCCCCCTTTCGCCAGCTGGCGTAATAGCGAAGAG
GCCCGCACCGATCGCCCTTCCCAACAGTTGCGCAGCCTGAATGGCGAATGGAAATT
GTAAGCGTTAATATTTTGTTAAAATTCGCGTTAAATTTTTGTTAAATCAGCTCATTTTT
TTAACCAATAGGCCGAAATCGGCAAAATCCCTTATAAATCAAAAGAATAGACCGAGA
TAGGGTTGAGTGTTGTTCCAGTTTGGAACAAGAGTCCACTATTAAGAACGTGGACT
CCAACGTCAAAGGGCGAAAAACCGTCTATCAGGGCGATGGCCCACTACGTGAACCA
TCACCCTAATCAAGTTTTTTGGGGTCGAGGTGCCGTAAAGCACTAAATCGGAACCCT
AAAGGGAGCCCCCGATTTAGAGCTTGACGGGGAAAGCCGGCGAACGTGGCGAGAA
AGGAAGGGAAGAAAGCGAAAGGAGCGGGCGCTAGGGCGCTGGCAAGTGTAGCGG
TCACGCTGCGCGTAACCACCACACCCGCCGCGCTTAATGCGCCGCTACAGGGCGCG
TCAGGTGGCACTTTTCGGGGAAATGTGCGCGGAACCCCTATTTGTTTATTTTTCTAAA
TACATTCAAATATGTATCCGCTCATGAGACAATAACCCTGATAAATGCTTCAATAATAT
TGAAAAAGGAAGAGTATGAGTATTCAACATTTCCGTGTCGCCCTTATTCCCTTTTTTG
CGGCATTTTGCCTTCCTGTTTTTGCTCACCCAGAAACGCTGGTGAAAGTAAAAGATG
CTGAAGATCAGTTGGGTGCACGAGTGGGTTACATCGAACTGGATCTCAACAGCGGT
AAGATCCTTGAGAGTTTTCGCCCCGAAGAACGTTTTCCAATGATGAGCACTTTTAAA
GTTCTGCTATGTGGCGCGGTATTATCCCGTATTGACGCCGGGCAAGAGCAACTCGGT
CGCCGCATACACTATTCTCAGAATGACTTGGTTGAGTACTCACCAGTCACAGAAAA
GCATCTTACGGATGGCATGACAGTAAGAGAATTATGCAGTGCTGCCATAACCATGAG
TGATAACACTGCGGCCAACTTACTTCTGACAACGATCGGAGGACCGAAGGAGCTAA
CCGCTTTTTTGCACAACATGGGGGATCATGTAACTCGCCTTGATCGTTGGGAACCGG
AGCTGAATGAAGCCATACCAAACGACGAGCGTGACACCACGATGCCTGTAGCAATG
GCAACAACGTTGCGCAAACTATTAACTGGCGAACTACTTACTCTAGCTTCCCGGCA
ACAATTAATAGACTGGATGGAGGCGGATAAAGTTGCAGGACCACTTCTGCGCTCGG
CCCTTCCGGCTGGCTGGTTTATTGCTGATAAATCTGGAGCCGGTGAGCGTGGGTCTC
GCGGTATCATTGCAGCACTGGGGCCAGATGGTAAGCCCTCCCGTATCGTAGTTATCT
ACACGACGGGGAGTCAGGCAACTATGGATGAACGAAATAGACAGATCGCTGAGATA
GGTGCCTCACTGATTAAGCATTGGTAACTGTCAGACCAAGTTTACTCATATATACTTT
AGATTGATTTAAAACTTCATTTTTAATTTAAAAGGATCTAGGTGAAGATCCTTTTTGA
TAATCTCATGACCAAAATCCCTTAACGTGAGTTTTCGTTCCACTGAGCGTCAGACCC
CGTAGAAAAGATCAAAGGATCTTCTTGAGATCCTTTTTTTCTGCGCGTAATCTGCTG

CTTGCAAACAAAAAAACCACCGCTACCAGCGGTGGTTTGTTTGCCGGATCAAGAGC
TACCAACTCTTTTTCCGAAGGTAACTGGCTTCAGCAGAGCGCAGATACCAAATACTG
TTCTTCTAGTGTAGCCGTAGTTAGGCCACCACTTCAAGAACTCTGTAGCACCGCCTA
CATACCTCGCTCTGCTAATCCTGTTACCAGTGGCTGCTGCCAGTGGCGATAAGTCGT
GTCTTACCGGGTTGGACTCAAGACGATAGTTACCGGATAAGGCGCAGCGGTCGGGC
TGAACGGGGGGTTCGTGCACACAGCCCAGCTTGGAGCGAACGACCTACACCGAAC
TGAGATACCTACAGCGTGAGCTATGAGAAAGCGCCACGCTTCCCGAAGGGAGAAA
GGCGGACAGGTATCCGGTAAGCGGCAGGGTCGGAACAGGAGAGCGCACGAGGGA
GCTTCCAGGGGGAAACGCCTGGTATCTTTATAGTCCTGTCGGGTTTCGCCACCTCTG
ACTTGAGCGTCGATTTTTGTGATGCTCGTCAGGGGGGCGGAGCCTATGGAAAAACG
CCAGCAACGCGGCCTTTTTACGGTTCCTGGCCTTTTGCTGGCCTTTTGCTCACATGT
TCTTTCCTGCGTTATCCCCTGATTCTGTGGATAACCGTATTACCGCCTTTGAGTGAGC
TGATACCGCTCGCCGCAGCCGAACGACCGAGCGCAGCGAGTCAGTGAGCGAGGAA
GCGGAAGAGCGCCCAATACGCAAACCGCCTCTCCCCGCGCGTTGGCCGATTCATTA
ATGCAGCTGGCACGACAGGTTTCCCGACTGGAAAGCGGGCAGTGAGCGCAACGCA
ATTAATGTGAGTTAGCTCACTCATTAGGCACCCCAGGCTTTACACTTTATGCTTCCGG
CTCGTATGTTGTGTGGAATTGTGAGCGGATAACAATTTCACACAGGAAACAGCTATG
ACCATGATTACGCCAA

### 2. Construction of AAV capsid protein expression plasmid encoding capsid protein of interest

The AAV capsid protein expression plasmid encoding the capsid protein of interest was constructed by the Gibson assembly method (the specific steps could be found in Gibson Assembly^{®} Chemical Transformation Protocol (E2611)). The PCR fragment was connected by Gibson assembly to the fragment of the intermediate plasmid RC2_IVB-NotI constructed in step 1 by linearization with the NotI enzyme to obtain different AAV capsid plasmids encoding the capsid protein of interest. The PCR fragments were obtained by template-free PCR, and the primers were paired themselves to perform PCR amplification. The primer sequences were shown in Table 2 below.

**Table 2:**

| Vector No. | PCR Primer |
|---|---|
| RC2_IVT1 | Forward primer (SEQ ID NO:30): |
| | AGCGGCTATTGTAGCCAGATCACCTCTGGCAGCAGCGTTGCCTCTCTGGAGGTT GGTAGA |
| | Reverse primer (SEQ ID NO:31): |
| | GCTGCTGCCAGAGGTGATCTGGCTACAATAGCCGCTAGACAAGCAGCTACCGC AGATGTC |
| RC2_IVT2 | Forward primer (SEQ ID NO:32): |
| | TGCAGCGAGGCGTCCCAAATCTCCTCTGGCTGCAGCGTTGCCTCTCTGGAGGTT GGTAGATAC |
| | Reverse primer (SEQ ID NO:33): |
| | GCTGCAGCCAGAGGAGATTTGGGACGCCTCGCTGCAAGACAAGCAGCTACCGC AGATGTCAAC |
| RC2_IVT3 | Forward primer (SEQ ID NO:34): |
| | TGCTGCAATATTCTGCAAATCACCTCGGGCAGCGGCGTTGCCTCTCTGGAGGTT GGTAGATACAG |
| | Reverse primer (SEQ ID NO:35): |
| | GCCGCTGCCCGAGGTGATTTGCAGAATATTGCAGCAAGACAAGCAGCTACCGC AGATGTCAACAC |
| RC2_IVT4 | Forward primer (SEQ ID NO:36): |
| | AGCTGCCAAATTCTGGAGGTCACCTCTTGCCGCAGCGTTGCCTCTCTGGAGGTT GGTAGATACAG |
| | Reverse primer (SEQ ID NO:37): |
| | GCTGCGGCAAGAGGTGACCTCCAGAATTTGGCAGCTAGACAAGCAGCTACCGC AGATGTCAACAC |
| RC2_IVT5 | Forward primer (SEQ ID NO:38): |
| | GGCGGCAATTGCTGCCAGATCACCTCTGGCTGCAGCGTTGCCTCTCTGGAGGTT GGTAGATACAG |
| | Reverse primer (SEQ ID NO:39): |
| | GCTGCAGCCAGAGGTGATCTGGCAGCAATTGCCGCCAGACAAGCAGCTACCGC AGATGTCAACAC |
| RC2_IVT6 | Forward primer (SEQ ID NO:40): |
| | CGCTGCCAGGGTTGCCAAGTCGCCTCTTGCAGCTGCGTTGCCTCTCTGGAGGTT GGTAGATACAG |
| | Reverse primer (SEQ ID NO:41): |
| | GCAGCTGCAAGAGGCGACTTGGCAACCCTGGCAGCGAGACAAGCAGCTACCG CAGATGTCAACACAC |
| RC2_IVT7 | Forward primer (SEQ ID NO:42): |
| | GGCGGCTGGCGTTGCCAAATCACCCCTAGCAGCCGCGTTGCCTCTCTGGAGGT TGGTAGATACAG |
| | Reverse primer (SEQ ID NO:43): |
| | GCGGCTGCTAGGGGTGATTTGGCAACGCCAGCCGCCAGACAAGCAGCTACCGC AGATGTCAACAC |
| RC2_IVT8 | Forward primer (SEQ ID NO:44): |
| | AGCTGCGGGTCTACCCAAATCACCGCGGGCTGCTGCGTTGCCTCTCTGGAGGTT GGTAGATACAG |
| | Reverse primer (SEQ ID NO:45): |
| | GCAGCAGCCCGCGGTGATTTGGGTAGACCCGCAGCTAGACAAGCAGCTACCGC AGATGTCAACAC |
| RC2_IVT9 | Forward primer (SEQ ID NO:46): |
| | CGCAGCAAGTGTACCAAGGTCTCCTCTGGCTGCTGCGTTGCCTCTCTGGAGGTT |
| | GGTAGATACAG |
| | Reverse primer (SEQ ID NO:47): |
| | GCAGCAGCCAGAGGAGACCTTGGTACACTTGCTGCGAGACAAGCAGCTACCGC AGATGTCAACACAC |
| RC2_IVT10 | Forward primer (SEQ ID NO:48): |
| | TGCTGCAATCCTTGCGAGGTCACCTCTTGCAGCCGCGTTGCCTCTCTGGAGGTT GGTAGATACAG |
| | Reverse primer (SEQ ID NO:49): |
| | GCGGCTGCAAGAGGTGACCTCGCAAGGATTGCAGCAAGACAAGCAGCTACCG CAGATGTCAACAC |
| RC2_IVT11 | Forward primer (SEQ ID NO:50): |
| | AGCGGCTATATTTTGCAGGCTGCCTCTCGCAGCAGCGTTGCCTCTCTGGAGGTT GGTAGATACAG |
| | Reverse primer (SEQ ID NO:51): |
| | GCTGCTGCGAGAGGCAGCCTGCAAAATATAGCCGCTAGACAAGCAGCTACCGC AGATGTCAACAC |
| RC2_IVT12 | Forward primer (SEQ ID NO:52): |
| | GGCAGCCAGATTTTGGAGAGATCCTCGTGCTGCTGCGTTGCCTCTCTGGAGGTT GGTAGATACAG |
| | Reverse primer (SEQ ID NO:53): |
| | GCAGCAGCACGAGGATCTCTCCAAAATCTGGCTGCCAGACAAGCAGCTACCGC AGATGTCAACAC |
| RC2_IVT13 | Forward primer (SEQ ID NO:54): |
| | TGCCGCAAGACTGCCCCTAGCAGCGGCGTTGCCTCTCTGGAGGTTGGTAGATAC AGAACCATACTG |
| | Reverse primer (SEQ ID NO:55): |
| | GCCGCTGCTAGGGGCAGTCTTGCGGCAAGACAAGCAGCTACCGCAGATGTCAA CACACAAGGCG |
| RC2_IVT14 | Forward primer (SEQ ID NO:56): |
| | CGGTAGCTGCTTGTCTGCCGTTTCTCTGGAGGTTGGTAGATACAG |
| | Reverse primer (SEQ ID NO:57): |
| | AACCTCCAGAGAAACGGCAGACAAGCAGCTACCGCAGATGTC |
| RC2_IVT15 | Forward primer (SEQ ID NO:58): |
| | GGCTGCTGCATGGGCCCGTCCGTTTCCTGCTGCTGCGTTGCCTCTCTGGAGGTT GGTAGATACAG |
| | Reverse primer (SEQ ID NO:59): |
| | GCAGCAGCAGGAAACGGACGGGCCCATGCAGCAGCCAGACAAGCAGCTACCG CAGATGTCAACAC |

Experimental results: In this example, the plasmid DNA to be constructed was identified by enzyme digestion and sanger sequencing, which confirmed successful construction of the expression plasmids of the corresponding capsid proteins. The amino acid sequences of the AAV capsid proteins expressed by the AAV capsid protein expression plasmids were specifically shown in Table 3 below.

**Table 3:**

| **Name and Amino Acid Sequence of Cap/AAV Capsid Protein** | **Sequence of Inserted or Substituted Targeting Peptide** | **Amino Acid Sequence of AAV Capsid Protein (Inserted or substituted targeting peptides shown in bold and underlined)** |
|---|---|---|
| IVT1 (SEQ ID NO: 15) | AAARGDLAT IAA (SEQ ID NO: 1) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLATIAARQ AATADVNTQGVLPGMVWQDRDVYLQGPIWAKIPHTD GHFHPSPLMGGFGLKHPPPQILIKNTPVPANPSTTFSAA KFASFITQYSTGQVSVEIEWELQKENSKRWNPEIQYTSN YNKSVNVDFTVDTNGVYSEPRPIGTRYLTRNL* |
| IVT2 (SEQ ID NO: 16) | AAARGDLGR LAA (SEQ ID NO: 2) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE |
| | | EIRTTNPVATEQYGSVSTNLQRGNAAARGDLGRLAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT3 (SEQ ID NO: 17) | AAARGDLQN IAA (SEQ ID NO: 3) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLQNIAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT4 (SEQ ID NO: 18) | AAARGDLQN LAA (SEQ ID NO: 4) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLQNLAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT5 (SEQ ID NO: 19) | AAARGDLAA IAA (SEQ ID NO: 5) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLAAIAARQ AATADVNTQGVLPGMVWQDRDVYLQGPIWAKIPHTD GHFHPSPLMGGFGLKHPPPQILIKNTPVPANPSTTFSAA KFASFITQYSTGQVSVEIEWELQKENSKRWNPEIQYTSN YNKSVNVDFTVDTNGVYSEPRPIGTRYLTRNL* |
| IVT6 (SEQ ID NO: 20) | AAARGDLAT LAA (SEQ ID NO: 6) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLATLAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT7 (SEQ ID NO: 21) | AAARGDLAT PAA (SEQ ID NO: 7) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP |
| | | LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLATPAARQ AATADVNTQGVLPGMVWQDRDVYLQGPIWAKIPHTD GHFHPSPLMGGFGLKHPPPQILIKNTPVPANPSTTFSAA KFASFITQYSTGQVSVEIEWELQKENSKRWNPEIQYTSN YNKSVNVDFTVDTNGVYSEPRPIGTRYLTRNL* |
| IVT8 (SEQ ID NO: 22) | AAARGDLGR PAA (SEQ ID NO: 8) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLGRPAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT9 (SEQ ID NO: 23) | AAARGDLGT LAA (SEQ ID NO: 9) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP |
| | | QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLGTLAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT10 (SEQ ID NO: 24) | AAARGDLAR IAA (SEQ ID NO: 10) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLARIAARQ AATADVNTQGVLPGMVWQDRDVYLQGPIWAKIPHTD GHFHPSPLMGGFGLKHPPPQILIKNTPVPANPSTTFSAA KFASFITQYSTGQVSVEIEWELQKENSKRWNPEIQYTSN YNKSVNVDFTVDTNGVYSEPRPIGTRYLTRNL* |
| IVT11 (SEQ ID NO: 25) | AAARGSLQN IAA (SEQ ID NO: 11) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE |
| | | EIRTTNPVATEQYGSVSTNLQRGNAAARGSLQNIAARQ AATADVNTQGVLPGMVWQDRDVYLQGPIWAKIPHTD GHFHPSPLMGGFGLKHPPPQILIKNTPVPANPSTTFSAA KFASFITQYSTGQVSVEIEWELQKENSKRWNPEIQYTSN YNKSVNVDFTVDTNGVYSEPRPIGTRYLTRNL* |
| IVT12 (SEQ ID NO:26) | AAARGSLQN LAA (SEQ ID NO: 12) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGSLQNLAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT13 (SEQ ID NO: 27) | AAARGSLAA (SEQ ID NO: 13) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGSLAARQAAT ADVNTQGVLPGMVWQDRDVYLQGPIWAKIPHTDGHF HPSPLMGGFGLKHPPPQILIKNTPVPANPSTTFSAAKFAS FITQYSTGQVSVEIEWELQKENSKRWNPEIQYTSNYNK SVNVDFTVDTNGVYSEPRPIGTRYLTRNL* |
| IVT14 (SEQ ID NO: 28) | NG | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRNGRQAATADVNTQGVL PGMVWQDRDVYLQGPIWAKIPHTDGHFHPSPLMGGFG LKHPPPQILIKNTPVPANPSTTFSAAKFASFITQYSTGQV SVEIEWELQKENSKRWNPEIQYTSNYNKSVNVDFTVDT NGVYSEPRPIGTRYLTRNL* |
| IVT15 (SEQ ID NO: 29) | AAAGNGRA HAAA (SEQ ID NO: 14) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAAGNGRAHAAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |

### Example 3: Production of AAV Viruses

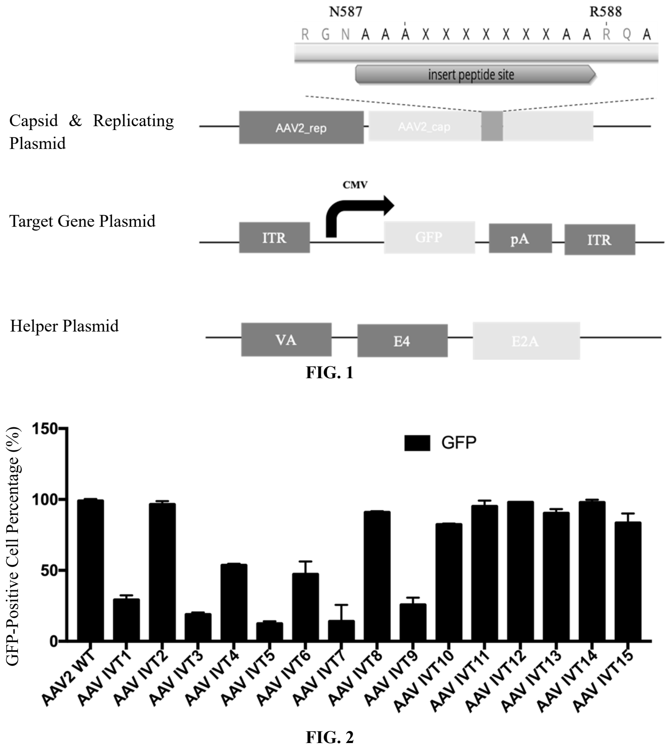

The packaging system for novel AAV viruses was a three-plasmid packaging system (FIG. 1). In this example, the three-plasmid co-transfection technique was employed to prepare recombinant AAVs. The three plasmids included RC plasmid (capsid & replicating plasmid; containing the nucleotide sequence encoding capsid Cap, *i.e.* the AAV capsid protein expression plasmids constructed in Example 2), Helper plasmid (or referred to as the Ad Helper plasmid or auxiliary plasmid, providing co-factors required for generating AAVs) and pAAV plasmid (or referred to as the transgene plasmid or target gene plasmid, which provided the nucleotide sequence to be delivered that generally contained the promoter, cDNA and the like required for protein translation). The AAV serotype was determined by the RC plasmid. For example, RC2 was used to package and prepare AAV2 serotype.

The HEK 293T cells were inoculated in a 100 mm TC-treated culture dish and grew until they reached 70% to 80% confluency for plasmid transfection. For AAV packaging, 5 µg of the AAV_GFP plasmid *(i.e.* GFP-expressing pAAV plasmids purchased from Cell Biolabs, Cat. No.: VPK-410-SER2), 5 µg of different AAV capsid protein expression plasmids encoding the capsid protein of interest constructed in Example 2 (and the AAV capsid protein expression plasmid encoding the wild-type capsid protein) and 10 µg of the Hepler plasmid (purchased from Cell Biolabs, Cat. No.: VPK-410-SER2) were co-transfected into the HEK 293T cells using the Poly ethylenimine Linear (PEI). Cells and supernatants were harvested 72 hours after transfection. The cells were pelleted by centrifugation at a low speed and lysed by adding 0.5% sodium deoxycholate (w/v). 50 U/ml Benzonase and 2 mM MgCl₂ were added, and incubation was carried out at 37°C for 2 hours to digest free DNA molecules, while pelleting the supernatant on ice with a one-fifth volume of a solution of 40% PEG8000 in 2.5M NaCl. The cell lysate and the supernatant were mixed and then centrifuged. The supernatant was measured and purified by the iodixanol ultracentrifugation purification method. The titers of the purified AAVs were determined by RT-qPCR and the purified AAVs were stored in a refrigerator at -80°C.

### Example 4: Experiment on In Vitro Cell Transduction with Novel AAVs

In order to test the *in vitro* transduction efficiency of novel AAVs (or novel AAV2) constructed in Example 3, different *GFP* gene-carrying AAVs (the capsid proteins expressed by the AAV capsid protein expression plasmids in AAVs were: IVT1/2/3/4/5/6/7/8/9/10/11/12/13/14/15, and the wild-type capsid protein) constructed in Example 3 were used.

Human retinal epitheliums (ARPE-19, Pricella, Cat. No.: CL-0026) were cultured under the following conditions: DMEM-F12 complete medium (89% DMEM-F12, 10% fetal bovine serum, and 1% penicillin-streptomycin). The cells were inoculated in a 24-well TC-treated culture plate at 1E+5 *(i.e.* 10⁵)/cell, and the AAV viruses of the novel capsids packaged in Example 3 and the wild-type AAV2 capsid (AAV2 WT) were added at a multiplicity of infection (MOI) of 3000. Three replicate wells were set for each type of virus. After the cells were infected with the virus, the culture plate was placed in a cell incubator (5% CO₂, 37°C) and cultured for 72 h. Subsequently, the cells were digested with 0.25% trypsin-EDTA (gibco, Cat. No.: 25200056). After the cells were re-suspended in PBS, the GFP fluorescence ratio was detected by flow cytometry (FIG. 2).

Referring to FIG. 2, the results showed that AAV IVT2/8/11/12/13/14/15 exhibited an *in vitro* infection efficiency in the target cell comparable to that of AAV2 WT.

### Example 5: Experiment on In Vivo Transduction of Novel AAVs in Mice

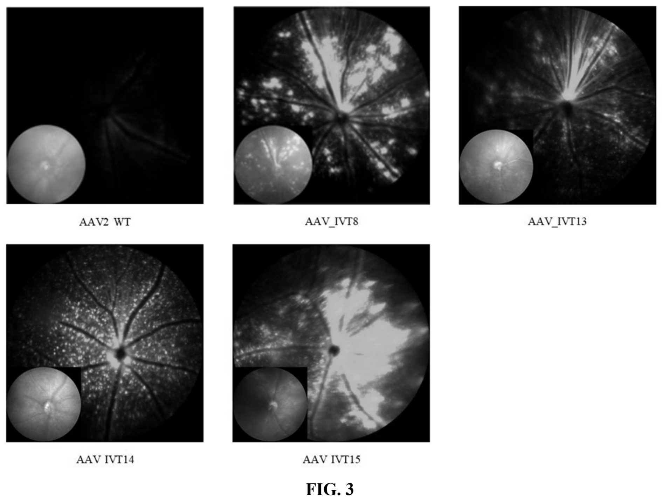

In order to test the *in vivo* transduction efficiency of the novel AAVs, 1 µL of virus particles of the *GFP* gene-carrying AAVs (AAV IVT8/13/14/15) or the wild-type AAV2 (AAV2 WT) (all the viruses used were prepared in Example 3) were injected at a titer of 1E+12 vg/mL (1E+9 vg/eye, *i.e.* 10⁹ vg/eye) via a microsyringe (Hamilton, 7633-01) into the vitreous cavities of 6-week-old C57BL/6 male mice (purchased from SPF (Beijing) Biotechnology Co., Ltd.). Two weeks after injection of viruses, the retinal fundi of mice were photographed by the Phoenix Micron IV retinal microscopy imaging system to acquire images of the GFP signals (FIG. 3).

Referring to FIG. 3, the results showed that AAV IVT8/13/15 had better infection efficiency in mouse retinae as compared to the wild-type AAV2.

### Example 6: Experiment on In Vitro Transduction of Novel AAVs in Human Retinal Organoids

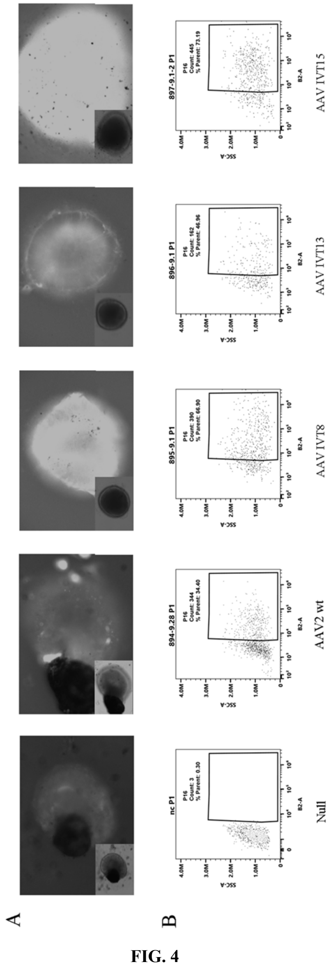

In order to test the ability of novel AAVs to target organoids, virus particles of the *GFP* gene-carrying AAVs (AAV IVT8/13/15) or the wild-type AAV2 (AAV2 WT) were added at 1E+10 vg (10¹⁰ vg)/organoid to human retinal organoids (constructed by Method 1 in Chichagova V, Hilgen G, Ghareeb A, Georgiou M, Carter M, Sernagor E, Lako M, Armstrong L. Human iPSC differentiation to retinal organoids in response to IGF1 and BMP4 activation is line- and method-dependent. Stem Cells. 2020 Feb;38(2):195-201. doi: 10.1002/stem.3116. Epub 2019 Dec 30. Erratum in: Stem Cells. 2020 Oct 1;38(10):E4. PMID: 31721366; PMCID: PMC7383896.). After infection with viruses for 72 h, images of GFP signals were acquired using a fluorescence microscope (A in FIG. 4). The organoids were digested with 0.25% trypsin-EDTA (gibco, Cat. No.: 25200056). The GFP fluorescence ratio was detected by flow cytometry (B in FIG. 4). In FIG. 4, Null indicated no transfection and wt indicated transfection of wild-type AAV2.

Referring to FIG. 4, the results showed that AAV IVT8/13/15 had better infection efficiency in retinal organoid as compared to the wild-type AAV2.

### Example 7: Experiment on In Vivo Transduction of Novel AAVs in Mice

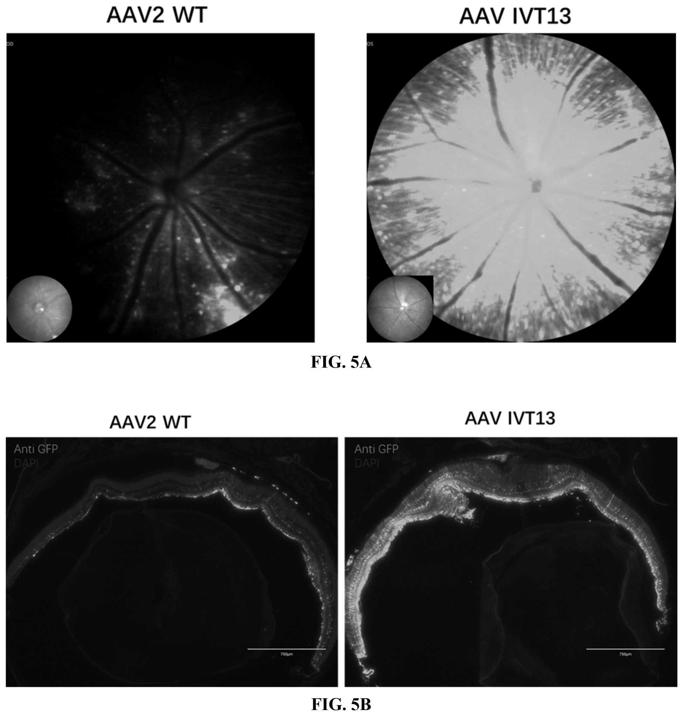

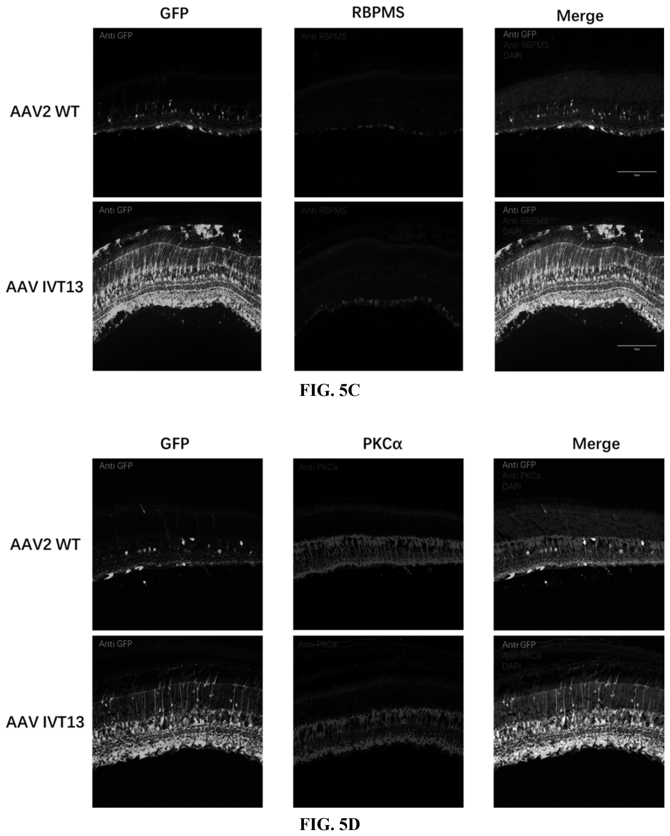

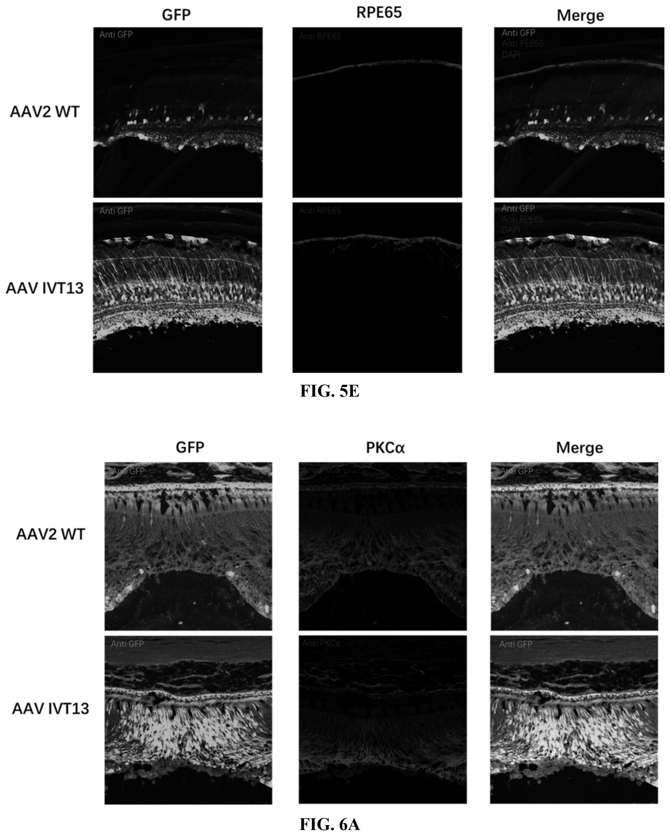

To test the *in vivo* transduction efficiency of the novel AAV IVT13 in mice, 1 µL of the virus particles of the *GFP* gene-carrying AAV IVT13 or the wild-type AAV2 (AAV2 WT) were injected at a titer of 2E+12 vg/mL (2E+9 vg/eye, *i.e.* 2×10⁹ vg/eye) via a microsyringe (Hamilton, 7633-01) into the vitreous cavities of 6-week-old C57BL/6 male mice (purchased from GemPharmatech Co., Ltd.). Three weeks after injection of viruses, the retinal fundi of mice were photographed by the Phoenix Micron IV retinal microscopy imaging system to acquire images of the GFP signals (FIG. 5A).

To test the position where the AAV IVT13 was transduced in the mouse retina, the mouse retinae were cryosectioned. The sections were immunofluorescently stained with antibodies against the retinal ganglion marker RBPMS (Abcam, ab152101, rabbit-derived antibody 1:500), the bipolar cell marker PKCα (Abcam, ab32376, rabbit-derived antibody 1:500), the retinal pigment epithelium marker RPE65 (Abcam, ab231782, rabbit-derived 1:500), and GFP (Invitrogen, A10262, chicken-derived antibody 1:500) respectively (FIG. 5B to FIG. 5E), and photographed by a Zeiss super-resolution confocal microscope ZEISS LSM880+ELYRAS.1 or a Thermo Fisher inverted fluorescence microscope EVOS-M5000.

The results showed that the GFP fluorescence level in the retina infected with AAV IVT13 was significantly higher than that in the retina infected with the wild-type AAV2 (FIG. 5A). In addition, the immunofluorescence results showed that despite intravitreal administration, the novel AAV IVT13 could diffuse from the ganglion layer to the photoreceptor layer and even the retinal pigment epithelium (RPE) (FIG. 5B to FIG. 5E), and was significantly better than the wild-type AAV2. As a result, the serotype AAV IVT13 exhibited stronger retinal tropism and transduction efficiency.

### Example 8: Experiment on In Vivo Transduction of Novel AAVs in Cynomolgus Monkeys

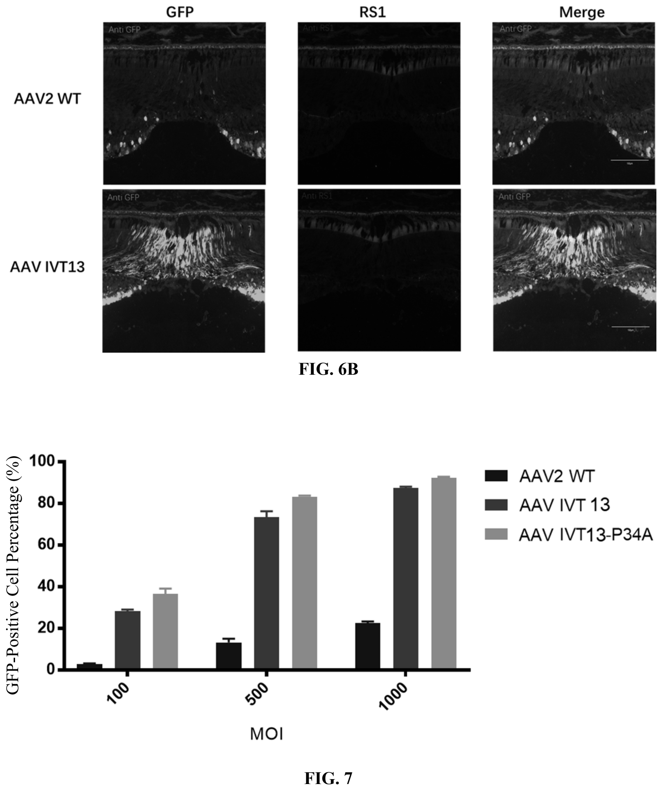

To test the *in vivo* transduction efficiency of novel AAV IVT13 in monkeys, two 5.7-year-old cynomolgus monkeys were anesthetized and intravitreally injected via a syringe (Wuxi Yushou Medical Instrument Co., Ltd., strength: 0.3 ml) with the virus particles of the *GFP* gene-carrying AAV IVT13 or the wild-type AAV2 (AAV2 WT) (the same virus was injected into both eyes of the same monkey at 1E+12 vg/eye, *i.e.* 10¹² vg/eye). This injection experiment was completed in TriApex Laboratories Co., Ltd. The retinae of monkeys were cryosectioned three weeks after injection of viruses. The sections were immunofluorescently stained with antibodies against the retinal bipolar cell marker PKCα (Abcam, ab32376, rabbit-derived antibody 1:500), the retinal photoreceptor layer marker RS1 (rabbit-derived antibody 1:500, abcam, ab314231), and GFP (Invitrogen, A10262, chicken-derived antibody 1:500) respectively (FIG. 6A and FIG. 6B), and photographed by a Zeiss super-resolution confocal microscope ZEISS LSM880+ELYRAS.1 or a Thermo Fisher inverted fluorescence microscope EVOS-M5000.

The results showed that the novel AAV IVT13 exhibited high tropism for the macular regions of monkeys. In addition, the IVT13 capsid enabled efficient transduction of the ganglion layer, the bipolar cells in the inner nuclear layer, and the photoreceptor cells in the outer nuclear layer of the retina (FIG. 6A and FIG. 6B), and its transduction efficiency is significantly better than that of the wild-type AAV2. Therefore, the serotype AAV IVT13 exhibited superior tropism and transduction efficiency in retinae of both mice and cynomolgus monkeys, and could deliver the gene to the layers of the retina.

### Example 9: Modification of AAV IVT13 Capsid Protein

In this example, modification was made to the RC2_IVT13 plasmid backbone by mutating the 34^{th} amino acid -- proline (P) to alanine (A) in the AAV2 capsid protein of this plasmid. General Biosystems (Anhui) Corp. Ltd. was commissioned to construct the plasmid. The specific sequence was as shown below (SEQ ID NO: 64):

TTGGTCTCTGCGAAGGGCGAATTCGTTTAAACCTGCAGGACTAGAGGTCCTGTA
TTAGAGGTCACGTGAGTGTTTTGCGACATTTTGCGACACCATGTGGTCACGCTGGGT
ATTTAAGCCCGAGTGAGCACGCAGGGTCTCCATTTTGAAGCGGGAGGTTTGAACGC
GCAGCCGCCAAGCCGAATTCTGCAGATATCCATCACACTGGCGGCCGCTCGACTAG
AGCGGCCGCCACCGCGGTGGAGCTCCAGCTTTTGTTCCCTTTAGTGAGGGTTAATT
GCGCGCTTGGCGTAATCATGGTCATAGCTGTTTCCTGTGTGAAATTGTTATCCGCTCA
CAATTCCACACAACATACGAGCCGGAAGCATAAAGTGTAAAGCCTGGGGTGCCTAA
TGAGTGAGCTAACTCACATTAATTGCGTTGCGCTCACTGCCCGCTTTCCAGTCGGGA
AACCTGTCGTGCCAGCTGCATTAATGAATCGGCCAACGCGCGGGGAGAGGCGGTTT
GCGTATTGGGCGCTCTTCCGCTTCCTCGCTCACTGACTCGCTGCGCTCGGTCGTTCG
GCTGCGGCGAGCGGTATCAGCTCACTCAAAGGCGGTAATACGGTTATCCACAGAAT
CAGGGGATAACGCAGGAAAGAACATGTGAGCAAAAGGCCAGCAAAAGGCCAGGA
ACCGTAAAAAGGCCGCGTTGCTGGCGTTTTTCCATAGGCTCCGCCCCCCTGACGAG
CATCACAAAAATCGACGCTCAAGTCAGAGGTGGCGAAACCCGACAGGACTATAAA

GATACCAGGCGTTTCCCCCTGGAAGCTCCCTCGTGCGCTCTCCTGTTCCGACCCTGC
CGCTTACCGGATACCTGTCCGCCTTTCTCCCTTCGGGAAGCGTGGCGCTTTCTCATA
GCTCACGCTGTAGGTATCTCAGTTCGGTGTAGGTCGTTCGCTCCAAGCTGGGCTGTG
TGCACGAACCCCCCGTTCAGCCCGACCGCTGCGCCTTATCCGGTAACTATCGTCTTG
AGTCCAACCCGGTAAGACACGACTTATCGCCACTGGCAGCAGCCACTGGTAACAGG
ATTAGCAGAGCGAGGTATGTAGGCGGTGCTACAGAGTTCTTGAAGTGGTGGCCTAA
CTACGGCTACACTAGAAGAACAGTATTTGGTATCTGCGCTCTGCTGAAGCCAGTTAC
CTTCGGAAAAAGAGTTGGTAGCTCTTGATCCGGCAAACAAACCACCGCTGGTAGCG
GTGGTTTTTTTGTTTGCAAGCAGCAGATTACGCGCAGAAAAAAAGGATCTCAAGAA
GATCCTTTGATCTTTTCTACGGGGTCTGACGCTCAGTGGAACGAAAACTCACGTTAA
GGGATTTTGGTCATGAGATTATCAAAAAGGATCTTCACCTAGATCCTTTTAAATTAAA
AATGAAGTTTTAAATCAATCTAAAGTATATATGAGTAAACTTGGTCTGACAGTTACCA
ATGCTTAATCAGTGAGGCACCTATCTCAGCGATCTGTCTATTTCGTTCATCCATAGTT
GCCTGACTCCCCGTCGTGTAGATAACTACGATACGGGAGGGCTTACCATCTGGCCCC
AGTGCTGCAATGATACCGCGAGACCCACGCTCACCGGCTCCAGATTTATCAGCAATA
AACCAGCCAGCCGGAAGGGCCGAGCGCAGAAGTGGTCCTGCAACTTTATCCGCCT
CCATCCAGTCTATTAATTGTTGCCGGGAAGCTAGAGTAAGTAGTTCGCCAGTTAATA
GTTTGCGCAACGTTGTTGCCATTGCTACAGGCATCGTGGTGTCACGCTCGTCGTTTG
GTATGGCTTCATTCAGCTCCGGTTCCCAACGATCAAGGCGAGTTACATGATCCCCCA
TGTTGTGCAAAAAAGCGGTTAGCTCCTTCGGTCCTCCGATCGTTGTCAGAAGTAAG
TTGGCCGCAGTGTTATCACTCATGGTTATGGCAGCACTGCATAATTCTCTTACTGTCA
TGCCATCCGTAAGATGCTTTTCTGTGACTGGTGAGTACTCAACCAAGTCATTCTGAG
AATAGTGTATGCGGCGACCGAGTTGCTCTTGCCCGGCGTCAATACGGGATAATACCG
CGCCACATAGCAGAACTTTAAAAGTGCTCATCATTGGAAAACGTTCTTCGGGGCGA
AAACTCTCAAGGATCTTACCGCTGTTGAGATCCAGTTCGATGTAACCCACTCGTGCA
CCCAACTGATCTTCAGCATCTTTTACTTTCACCAGCGTTTCTGGGTGAGCAAAAACA
GGAAGGCAAAATGCCGCAAAAAAGGGAATAAGGGCGACACGGAAATGTTGAATAC
TCATACTCTTCCTTTTTCAATATTATTGAAGCATTTATCAGGGTTATTGTCTCATGAGC
GGATACATATTTGAATGTATTTAGAAAAATAAACAAATAGGGGTTCCGCGCACATTTC
CCCGAAAAGTGCCACCTAAATTGTAAGCGTTAATATTTTGTTAAAATTCGCGTTAAAT
TTTTGTTAAATCAGCTCATTTTTTAACCAATAGGCCGAAATCGGCAAAATCCCTTATA
AATCAAAAGAATAGACCGAGATAGGGTTGAGTGTTGTTCCAGTTTGGAACAAGAGT
CCACTATTAAAGAACGTGGACTCCAACGTCAAAGGGCGAAAAACCGTCTATCAGGG
CGATGGCCCACTACGTGAACCATCACCCTAATCAAGTTTTTTGGGGTCGAGGTGCCG
TAAAGCACTAAATCGGAACCCTAAAGGGAGCCCCCGATTTAGAGCTTGACGGGGAA

AGCCGGCGAACGTGGCGAGAAAGGAAGGGAAGAAAGCGAAAGGAGCGGGCGCTA
GGGCGCTGGCAAGTGTAGCGGTCACGCTGCGCGTAACCACCACACCCGCCGCGCTT
AATGCGCCGCTACAGGGCGCGTCCCATTCGCCATTCAGGCTGCGCAACTGTTGGGA
AGGGCGATCGGTGCGGGCCTCTTCGCTATTACGCCAGCTGGCGAAAGGGGGATGTG
CTGCAAGGCGATTAAGTTGGGTAACGCCAGGGTTTTCCCAGTCACGACGTTGTAAA
ACGACGGCCAGTGAGCGCGCGTAATACGACTCACTATAGGGCGAATTGGGTACCGG
GCCCCCCCTCGATCGAGGTCGACGGTATCGGGGGAGCTCGCAGGGTCTCCATTTTG
AAGCGGGAGGTTTGAACGCGCAGCCGCCATGCCGGGGTTTTACGAGATTGTGATTA
AGGTCCCCAGCGACCTTGACGAGCATCTGCCCGGCATTTCTGACAGCTTTGTGAAC
TGGGTGGCCGAGAAGGAATGGGAGTTGCCGCCAGATTCTGACATGGATCTGAATCT
GATTGAGCAGGCACCCCTGACCGTGGCCGAGAAGCTGCAGCGCGACTTTCTGACG
GAATGGCGCCGTGTGAGTAAGGCCCCGGAGGCCCTTTTCTTTGTGCAATTTGAGAA
GGGAGAGAGCTACTTCCACATGCACGTGCTCGTGGAAACCACCGGGGTGAAATCCA
TGGTTTTGGGACGTTTCCTGAGTCAGATTCGCGAAAAACTGATTCAGAGAATTTACC
GCGGGATCGAGCCGACTTTGCCAAACTGGTTCGCGGTCACAAAGACCAGAAATGG
CGCCGGAGGCGGGAACAAGGTGGTGGATGAGTGCTACATCCCCAATTACTTGCTCC
CCAAAACCCAGCCTGAGCTCCAGTGGGCGTGGACTAATATGGAACAGTATTTAAGC
GCCTGTTTGAATCTCACGGAGCGTAAACGGTTGGTGGCGCAGCATCTGACGCACGT
GTCGCAGACGCAGGAGCAGAACAAAGAGAATCAGAATCCCAATTCTGATGCGCCG
GTGATCAGATCAAAAACTTCAGCCAGGTACATGGAGCTGGTCGGGTGGCTCGTGGA
CAAGGGGATTACCTCGGAGAAGCAGTGGATCCAGGAGGACCAGGCCTCATACATCT
CCTTCAATGCGGCCTCCAACTCGCGGTCCCAAATCAAGGCTGCCTTGGACAATGCG
GGAAAGATTATGAGCCTGACTAAAACCGCCCCCGACTACCTGGTGGGCCAGCAGCC
CGTGGAGGACATTTCCAGCAATCGGATTTATAAAATTTTGGAACTAAACGGGTACGA
TCCCCAATATGCGGCTTCCGTCTTTCTGGGATGGGCCACGAAAAAGTTCGGCAAGA
GGAACACCATCTGGCTGTTTGGGCCTGCAACTACCGGGAAGACCAACATCGCGGAG
GCCATAGCCCACACTGTGCCCTTCTACGGGTGCGTAAACTGGACCAATGAGAACTT
TCCCTTCAACGACTGTGTCGACAAGATGGTGATCTGGTGGGAGGAGGGGAAGATG
ACCGCCAAGGTCGTGGAGTCGGCCAAAGCCATTCTCGGAGGAAGCAAGGTGCGCG
TGGACCAGAAATGCAAGTCCTCGGCCCAGATAGACCCGACTCCCGTGATCGTCACC
TCCAACACCAACATGTGCGCCGTGATTGACGGGAACTCAACGACCTTCGAACACCA
GCAGCCGTTGCAAGACCGGATGTTCAAATTTGAACTCACCCGCCGTCTGGATCATG
ACTTTGGGAAGGTCACCAAGCAGGAAGTCAAAGACTTTTTCCGGTGGGCAAAGGA
TCACGTGGTTGAGGTGGAGCATGAATTCTACGTCAAAAAGGGTGGAGCCAAGAAA
AGACCCGCCCCCAGTGACGCAGATATAAGTGAGCCCAAACGGGTGCGCGAGTCAG

TTGCGCAGCCATCGACGTCAGACGCGGAAGCTTCGATCAACTACGCAGACAGGTAC
CAAAACAAATGTTCTCGTCACGTGGGCATGAATCTGATGCTGTTTCCCTGCAGACAA
TGCGAGAGAATGAATCAGAATTCAAATATCTGCTTCACTCACGGACAGAAAGACTG
TTTAGAGTGCTTTCCCGTGTCAGAATCTCAACCCGTTTCTGTCGTCAAAAAGGCGTA
TCAGAAACTGTGCTACATTCATCATATCATGGGAAAGGTGCCAGACGCTTGCACTGC
CTGCGATCTGGTCAATGTGGATTTGGATGACTGCATCTTTGAACAATAAATGATTTAA
ATCAGGTATGGCTGCCGATGGTTATCTTCCAGATTGGCTCGAGGACACTCTCTCTGA
AGGAATAAGACAGTGGTGGAAGCTCAAACCTGGCCCACCACCACCAAAGGCCGCA
GAGCGGCATAAGGACGACAGCAGGGGTCTTGTGCTTCCTGGGTACAAGTACCTCGG
ACCCTTCAACGGACTCGACAAGGGAGAGCCGGTCAACGAGGCAGACGCCGCGGCC
CTCGAGCACGACAAAGCCTACGACCGGCAGCTCGACAGCGGAGACAACCCGTACC
TCAAGTACAACCACGCCGACGCGGAGTTTCAGGAGCGCCTTAAAGAAGATACGTCT
TTTGGGGGCAACCTCGGACGAGCAGTCTTCCAGGCGAAAAAGAGGGTTCTTGAAC
CTCTGGGCCTGGTTGAGGAACCTGTTAAGACGGCTCCGGGAAAAAAGAGGCCGGT
AGAGCACTCTCCTGTGGAGCCAGACTCCTCCTCGGGAACCGGAAAGGCGGGCCAG
CAGCCTGCAAGAAAAAGATTGAATTTTGGTCAGACTGGAGACGCAGACTCAGTACC
TGACCCCCAGCCTCTCGGACAGCCACCAGCAGCCCCCTCTGGTCTGGGAACTAATA
CGATGGCTACAGGCAGTGGCGCACCAATGGCAGACAATAACGAGGGCGCCGACGG
AGTGGGTAATTCCTCGGGAAATTGGCATTGCGATTCCACATGGATGGGCGACAGAG
TCATCACCACCAGCACCCGAACCTGGGCCCTGCCCACCTACAACAACCACCTCTAC
AAACAAATTTCCAGCCAATCAGGAGCCTCGAACGACAATCACTACTTTGGCTACAG
CACCCCTTGGGGGTATTTTGACTTCAACAGATTCCACTGCCACTTTTCACCACGTGA
CTGGCAAAGACTCATCAACAACAACTGGGGATTCCGACCCAAGAGACTCAACTTC
AAGCTCTTTAACATTCAAGTCAAAGAGGTCACGCAGAATGACGGTACGACGACGAT
TGCCAATAACCTTACCAGCACGGTTCAGGTGTTTACTGACTCGGAGTACCAGCTCCC
GTACGTCCTCGGCTCGGCGCATCAAGGATGCCTCCCGCCGTTCCCAGCAGACGTCT
TCATGGTGCCACAGTATGGATACCTCACCCTGAACAACGGGAGTCAGGCAGTAGGA
CGCTCTTCATTTTACTGCCTGGAGTACTTTCCTTCTCAGATGCTGCGTACCGGAAAC
AACTTTACCTTCAGCTACACTTTTGAGGACGTTCCTTTCCACAGCAGCTACGCTCAC
AGCCAGAGTCTGGACCGTCTCATGAATCCTCTCATCGACCAGTACCTGTATTACTTG
AGCAGAACAAACACTCCAAGTGGAACCACCACGCAGTCAAGGCTTCAGTTTTCTC
AGGCCGGAGCGAGTGACATTCGGGACCAGTCTAGGAACTGGCTTCCTGGACCCTGT
TACCGCCAGCAGCGAGTATCAAAGACATCTGCGGATAACAACAACAGTGAATACTC
GTGGACTGGAGCTACCAAGTACCACCTCAATGGCAGAGACTCTCTGGTGAATCCGG
GCCCGGCCATGGCAAGCCACAAGGACGATGAAGAAAAGTTTTTTCCTCAGAGCGG

GGTTCTCATCTTTGGGAAGCAAGGCTCAGAGAAAACAAATGTGGACATTGAAAAG
GTCATGATTACAGACGAAGAGGAAATCAGGACAACCAATCCCGTGGCTACGGAGCA
GTATGGTTCTGTATCTACCAACCTCCAGAGAGGCAACGCCGCTGCTAGGGGCAGTC
TTGCGGCAAGACAAGCAGCTACCGCAGATGTCAACACACAAGGCGTTCTTCCAGG
CATGGTCTGGCAGGACAGAGATGTGTACCTTCAGGGGCCCATCTGGGCAAAGATTC
CACACACGGACGGACATTTTCACCCCTCTCCCCTCATGGGTGGATTCGGACTTAAAC
ACCCTCCTCCACAGATTCTCATCAAGAACACCCCGGTACCTGCGAATCCTTCGACCA
CCTTCAGTGCGGCAAAGTTTGCTTCCTTCATCACACAGTACTCCACGGGACAGGTC
AGCGTGGAGATCGAGTGGGAGCTGCAGAAGGAAAACAGCAAACGCTGGAATCCCG
AAATTCAGTACACTTCCAACTACAACAAGTCTGTTAATGTGGACTTTACTGTGGACA
CTAATGGCGTGTATTCAGAGCCTCGCCCCATTGGCACCAGATACCTGACTCGTAATC
TGTAATTGCCTGTTAATCAATAAACCGGTTGATTCGTTTCAGTTGAACT

The amino acid sequence of the AAV capsid protein IVT13-P34A expressed by the resulting AAV capsid protein expression plasmid was as shown below (SEQ ID NO: 65):

MAADGYLPDWLEDTLSEGIRQWWKLKPGPPPPKAAERHKDDSRGLVLPGYKYL
GPFNGLDKGEPVNEADAAALEHDKAYDRQLDSGDNPYLKYNHADAEFQERLKEDTS
FGGNLGRAVFQAKKRVLEPLGLVEEPVKTAPGKKRPVEHSPVEPDSSSGTGKAGQQPA
RKRLNFGQTGDADSVPDPQPLGQPPAAPSGLGTNTMATGSGAPMADNNEGADGVGN
SSGNWHCDSTWMGDRVITTSTRTWALPTYNNHLYKQISSQSGASNDNHYFGYSTPWG
YFDFNRFHCHFSPRDWQRLINNNWGFRPKRLNFKLFNIQVKEVTQNDGTTTIANNLTS
TVQVFTDSEYQLPYVLGSAHQGCLPPFPADVFMVPQYGYLTLNNGSQAVGRSSFYCLE
YFPSQMLRTGNNFTFSYTFEDVPFHSSYAHSQSLDRLMNPLIDQYLYYLSRTNTPSGTT
TQSRLQFSQAGASDIRDQSRNWLPGPCYRQQRVSKTSADNNNSEYSWTGATKYHLNG
RDSLVNPGPAMASHKDDEEKFFPQSGVLIFGKQGSEKTNVDIEKVMITDEEEIRTTNPVA
TEQYGSVSTNLQRGNAAARGSLAARQAATADVNTQGVLPGMVWQDRDVYLQGPIWA
KIPHTDGHFHPSPLMGGFGLKHPPPQILIKNTPVPANPSTTFSAAKFASFITQYSTGQVSV
EIEWELQKENSKRWNPEIQYTSNYNKSVNVDFTVDTNGVYSEPRPIGTRYLTRNL*

In the above sequence, the mutation site of IVT13-P34A relative to the IVT13 capsid protein was shown in bold and underlined; and the targeting peptide 13 was shown in italics and underlined.

### Example 10: Experiment on In Vitro Transduction of AAV IVT13-P34A

To test the *in vitro* transduction efficiency of the IVT13-P34A capsid protein constructed in Example 9, the method of Example 3 was followed to prepare *GFP* gene-carrying AAVs (AAV2 WT, AAV IVT13, and AAV IVT13-P34A), and these *GFP* gene-carrying AAVs (AAV2 WT, AAV IVT13, and AAV IVT13-P34A) were used to infect human retinal epitheliums (ARPE-19) to test their transduction efficiencies.

Human retinal epitheliums (ARPE-19, Pricella, Cat. No.: CL-0026) were cultured under the following conditions: DMEM-F12 complete medium (89% DMEM-F12, 10% fetal bovine serum, and 1% penicillin-streptomycin). The cells were inoculated in a 24-well TC-treated culture plate at 1E+5/cell, and the AAV viruses of the novel capsids (IVT13 and IVT13-P34A) packaged by the method of Example 3 and the wild-type AAV2 capsid (AAV2 WT) were added at a multiplicity of infection (MOI) of 100/500/1000. Three replicate wells were set for each type of virus. After the cells were infected with the virus, the culture plate was placed in a cell incubator (5% CO₂, 37°C) and cultured for 48 h. Subsequently, the cells were digested with 0.25% trypsin-EDTA (gibco, Cat. No.: 25200056). After the cells were re-suspended in PBS, the GFP fluorescence ratio was detected by flow cytometry (FIG. 7).

Referring to FIG. 7 and Table 4 below, the results showed that when the ARPE cells were infected with different MOIs, the transduction efficiency of the modified AAV IVT13-P34A was significantly enhanced as compared to the wild-type AAV2 and also improved as compared to the AAV IVT13.

**Table 4:**

| MOI | AAV2 WT | | | AAV IVT13 | | | AAV IVT13-P34A | | |
|---|---|---|---|---|---|---|---|---|---|
| MOI=100 | 3.18 | 2.69 | 3.01 | 29.1 | 28.07 | 27.99 | 39.12 | 36.63 | 33.96 |
| MOI=500 | 11.58 | 13.36 | 15.01 | 70.98 | 73.02 | 76.48 | 83.25 | 83.79 | 82.89 |
| MOI=1000 | 23.36 | 21.69 | 22.53 | 87.34 | 87.12 | 88.22 | 92.01 | 91.87 | 93 |

In Table 4 above, the values indicated the percentage (unit: %) of the GFP-positive cells.

### Example 11: Experiment on In Vitro Transduction of AAV IVT13-P34A in Human Retinal Organoids

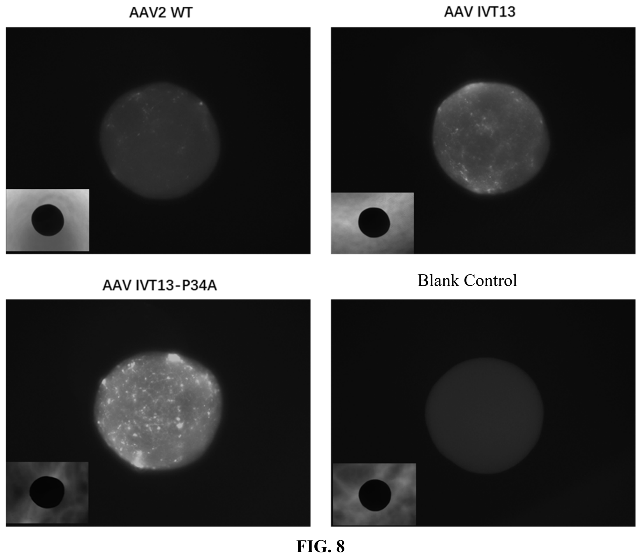

To test the targeting effect of AAV IVT13-P34A towards organoids, the *GFP* gene-carrying AAV viruses (AAV2 WT, AAV IVT13, and AAV IVT13-P34A) were added at 1E+10 vg/organoid to human retinal organoids harvested on day 79 of differentiation (constructed by Method 1 in Chichagova V, Hilgen G, Ghareeb A, Georgiou M, Carter M, Sernagor E, Lako M, Armstrong L. Human iPSC differentiation to retinal organoids in response to IGF1 and BMP4 activation is line- and method-dependent. Stem Cells. 2020 Feb;38(2):195-201. doi: 10.1002/stem.3116. Epub 2019 Dec 30. Erratum in: Stem Cells. 2020 Oct 1;38(10):E4. PMID: 31721366; PMCID: PMC7383896.), and the blank control did not receive viral infection. Eight days after infection, photographs were taken using a Thermo Fisher inverted fluorescence microscope (EVOS-M5000) to acquire the images of GFP signals (FIG. 8).

Referring to FIG. 8, the results showed that the AAV IVT13-P34A had better ability to infect retinal organoids as compared to the wild-type AAV2 (AAV2 WT) and AAV IVT13.

## Claims

1. An adeno-associated virus capsid protein, comprising a targeting peptide, wherein the targeting peptide comprises one or more of the following sequences:
(i) a sequence AAX₁X₂X₃X₄X₅X₆X₇X₈AA; and
(ii) a sequence NG;
wherein in the sequence AAX₁X₂X₃X₄X₅X₆X₇X₈AA:
(a) X₁ is A;
(b) X₂ is R or G;
(c) X₃ is G or N;
(d) X₄ is D, S or G;
(e) X₅ is L or R;
(f) X₆ is A, G, Q or a deletion;
(g) X₇ is T, R, N, A, H or a deletion; and
(h) X₈ is I, L, P, A or a deletion.

2. The adeno-associated virus capsid protein according to claim 1, wherein in the sequence AAX₁X₂X₃X₄X₅X₆X₇X₈AA:
(a) X₁ is A;
(b) X₂ is R;
(c) X₃ is G;
(d) X₄ is D or S;
(e) X₅ is L;
(f) X₆ is A, G, Q or a deletion;
(g) X₇ is T, R, N, A or a deletion; and
(h) X₈ is I, L, P or a deletion.

3. The adeno-associated virus capsid protein according to claim 1 or 2, wherein the targeting peptide comprises an amino acid sequence as set forth in any one of SEQ ID NOs: 1 to 14 and the sequence NG;
preferably, the targeting peptide comprises an amino acid sequence as set forth in any one of SEQ ID NOs: 2, 8, 11, 12, 13, and 14 and the sequence NG; and
more preferably, the targeting peptide comprises an amino acid sequence as set forth in any one of SEQ ID NOs: 8, 13, and 14 and the sequence NG.

4. The adeno-associated virus capsid protein according to any one of claims 1 to 3, wherein the adeno-associated virus is one or more selected from AAV1, AAV2, AAV3, AAV4, AAV5, AAV6, AAV7, AAV8, AAV9, rh.10, rh.39, rh.43, and rh.74;
preferably, AAV comprises AAV2.

5. The adeno-associated virus capsid protein according to any one of claims 1 to 4, wherein AAV2 comprises AAV2 VP1 or a variant thereof;
preferably, the amino acid sequence of AAV2 VP1 is as set forth in SEQ ID NO: 60;
preferably, the variant of AAV2 VP1 comprises a variant in which proline (P) at position 34 is mutated to alanine (A) relative to the amino acid sequence as set forth in SEQ ID NO: 60.

6. The adeno-associated virus capsid protein according to any one of claims 1 to 5, wherein the targeting peptide is inserted at a position corresponding to the 587^{th} amino acid in AAV2 VP1 or a variant thereof; or
the targeting peptide substitutes for the 586^{th} amino acid and the 587^{th} amino acid in AAV2 VP1 or a variant thereof;
preferably, the adeno-associated virus capsid protein comprises an amino acid sequence as set forth in any one of SEQ ID NOs: 15 to 29 and 65, or an amino acid sequence having at least 80%, 85%, 90%, 95%, 97% or 99% identity to the amino acid sequence as set forth in any one of SEQ ID NOs: 15 to 29 and 65.

7. A targeting peptide, comprising one or more of the following sequences:
(i) a sequence AAX₁X₂X₃X₄X₅X₆X₇X₈AA; and
(ii) a sequence NG;
wherein in the sequence AAX₁X₂X₃X₄X₅X₆X₇X₈AA:
(a) X₁ is A;
(b) X₂ is R or G;
(c) X₃ is G or N;
(d) X₄ is D, S or G;
(e) X₅ is L or R;
(f) X₆ is A, G, Q or a deletion;
(g) X₇ is T, R, N, A, H or a deletion; and
(h) X₈ is I, L, P, A or a deletion;
optionally, in the sequence AAX₁X₂X₃X₄X₅X₆X₇X₈AA:
(a) X₁ is A;
(b) X₂ is R;
(c) X₃ is G;
(d) X₄ is D or S;
(e) X₅ is L;
(f) X₆ is A, G, Q or a deletion;
(g) X₇ is T, R, N, A or a deletion; and
(h) X₈ is I, L, P or a deletion;
preferably, the targeting peptide comprises an amino acid sequence as set forth in any one of SEQ ID NOs: 1 to 14 and the sequence NG;
more preferably, the targeting peptide comprises an amino acid sequence as set forth in any one of SEQ ID NOs: 2, 8, 11, 12, 13, and 14 and the sequence NG;
further preferably, the targeting peptide comprises an amino acid sequence as set forth in any one of SEQ ID NOs: 8, 13 and 14.

8. A polynucleotide encoding the adeno-associated virus capsid protein according to any one of claims 1 to 6, or encoding the targeting peptide according to claim 7.

9. An adeno-associated virus, comprising the adeno-associated virus capsid protein according to any one of claims 1 to 6.

10. The adeno-associated virus according to claim 9, further comprising a transgene; optionally, the transgene being a therapeutic transgene, a prophylactic transgene or a diagnostic transgene.

11. A transgene delivery vector, comprising the adeno-associated virus according to claim 9 or 10.

12. A pharmaceutical composition, comprising:
(a) the adeno-associated virus according to claim 9 or 10 or the transgene delivery vector according to claim 11; and optionally
(b) a pharmaceutically acceptable carrier.

13. Use of the adeno-associated virus according to claim 9 or 10, the transgene delivery vector according to claim 11, or the pharmaceutical composition according to claim 12 in preparation of a reagent for delivering a transgene to a cell.

14. The use according to claim 13, wherein the cell is derived from a subject;
preferably, the subject is a mammalian subject;
more preferably, the subject is a human.

15. The use according to claim 13 or 14, wherein the cell is derived from an eye.

16. The use according to claim 14 or 15, wherein the subject suffers from an eye disease.

17. The use according to any one of claims 13 to 16, wherein the adeno-associated virus, the transgene delivery vector, or the pharmaceutical composition is administered to a subject via subretinal or intravitreal injection.

18. Use of the adeno-associated virus according to claim 9 or 10, the transgene delivery vector according to claim 11, or the pharmaceutical composition according to claim 12 in preparation of a medicament for treating a disease;
preferably, the disease comprises an eye disease.

19. A method for treating a disease, comprising a step of administering, to a subject, the adeno-associated virus according to claim 9 or 10, the transgene delivery vector according to claim 11, or the pharmaceutical composition according to claim 12;
preferably, the disease comprising an eye disease.
